# EUROPEAN PATENT SPECIFICATION

(11) **EP 3 968 994 B1**
(45) Date of publication and mention of the grant of the patent: **27.12.2023**
(21) Application number: 20729547.8
(22) Date of filing: 13.05.2020
(51) Int. Cl.: A61K 31/4545, A61P 25/00, A61P 25/28, A61P 29/00, A61P 35/00, A61P 37/04, C07D 401/14, C07D 405/14

(54) **ABHD12 INHIBITORS AND METHODS OF MAKING AND USING SAME**
ABHD12-INHIBITOREN UND VERFAHREN ZUR HERSTELLUNG UND VERWENDUNG DAVON
INHIBITEURS ABHD12 ET LEURS PROCÉDÉS DE FABRICATION ET D'UTILISATION

(30) Priority: 14.05.2019 US 201962847804 P
(43) Date of publication of application: 23.03.2022
(73) Proprietor: H. Lundbeck A/S, 2500 Valby (DK)
(72) Inventor: GRICE, Cheryl A., San Diego, California 92121 (US); MOODY, Jeanne V., San Diego, California 92121 (US); BUZARD, Daniel J., San Diego, California 92121 (US); CISAR, Justin S., San Diego, California 92121 (US)
(74) Representative: H. Lundbeck A/S
(86) International application number: PCT/US2020/032721
(87) International publication number: WO 2020/232153

(56) References cited:
- WO-A1-2019/222267
- US-A1- 2013 331 396
- DAISUKE OGASAWARA ET AL: "Discovery and Optimization of Selective and in Vivo Active Inhibitors of the Lysophosphatidylserine Lipase [alpha]/[beta]-Hydrolase Domain-Containing 12 (ABHD12)", JOURNAL OF MEDICINAL CHEMISTRY, vol. 62, no. 3, 5 February 2019 (2019-02-05), pages 1643-1656, XP055707531, US ISSN: 0022-2623, DOI: 10.1021/acs.jmedchem.8b01958
- OGASAWARA DAISUKE ET AL: "Selective blockade of the lyso-PS lipase ABHD12 stimulates immune responses in vivo", NATURE CHEMICAL BIOLOGY, NATURE PUBLISHING GROUP, BASINGSTOKE, vol. 14, no. 12, 12 November 2018 (2018-11-12), pages 1099-1108, XP036637462, ISSN: 1552-4450, DOI: 10.1038/S41589-018-0155-8 [retrieved on 2018-11-12]

## Description

### FIELD OF THE INVENTION

The present invention relates to compounds which are ABHD12 inhibitors.

### BACKGROUND

Alpha/beta-hydrolase domain containing 12 (ABHD12) is a serine hydrolase encoded by the ABHD12 gene that participates in the breakdown of the endocannabinoid neurotransmitter 2-arachidonylglycerol (2-AG) in the central nervous system.

Ogasawara et al., Journal of medicinal chemistry, vol 62, no. 3, February 2019, 1643-1656 discloses potent and selective ABHD12 inhibitors.

Ogasawara et al., Nature chemical biology, vol. 14, no. 12, November 2018, 1099-1108 disclsoses thiourea ABHD12 inhibitors.

US 2013/331396 discloses piperazinyl and piperidinyl urea compounds are useful as FAAH inhibitors.

WO2019/222267 discloses compounds and compositions useful as modulators of ABHD12.

### BRIEF SUMMARY OF THE INVENTION

This disclosure provides, for example, compounds and compositions which are modulators of ABHD12, and their use as medicinal agents, processes for their preparation, and pharmaceutical compositions that include disclosed compounds as at least one active ingredient. The disclosure also provides for the use of disclosed compounds as medicaments and/or in the manufacture of medicaments for the inhibition of ABHD12 activity in warm-blooded animals such as humans.
one aspect according to the invention is a compound selected from: 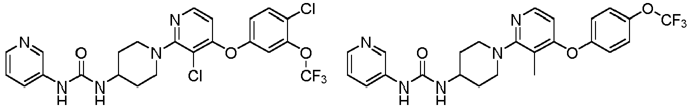 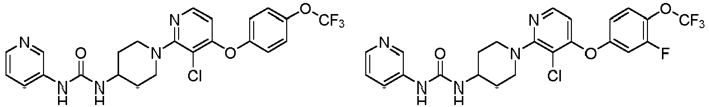 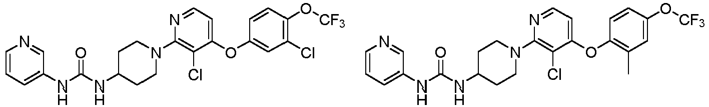 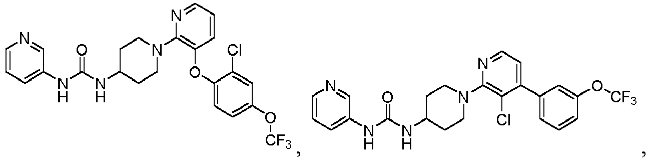 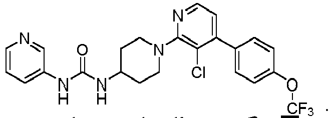 or a pharmacheutically acceptable salt thereof.

another embodiment according to the invention is a pharmaceutical composition comprising a compound described herein, or a pharmaceutically acceptable salt thereof, and at least one pharmaceutically acceptable excipient.

### DETAILED DESCRIPTION OF THE INVENTION

This disclosure is directed, at least in part, to modulators or inhibitors of ABHD12. For example, provided herein are compounds capable of inhibiting ABHD12.

As used herein and in the appended claims, the singular forms "a," "and," and "the" include plural referents unless the context clearly dictates otherwise. Thus, for example, reference to "an agent" includes a plurality of such agents, and reference to "the cell" includes reference to one or more cells (or to a plurality of cells) and equivalents thereof. When ranges are used herein for physical properties, such as molecular weight, or chemical properties, such as chemical formulae, all combinations and subcombinations of ranges and specific embodiments therein are intended to be included. The term "about" when referring to a number or a numerical range means that the number or numerical range referred to is an approximation within experimental variability (or within statistical experimental error), and thus the number or numerical range varies between 1% and 15% of the stated number or numerical range. The term "comprising" (and related terms such as "comprise" or "comprises" or "having" or "including") is not intended to exclude that which in other certain embodiments, for example, an embodiment of any composition of matter, composition, method, or process, or the like, described herein, may "consist of" or "consist essentially of" the described features.

### Definitions

As used in the specification and appended claims, unless specified to the contrary, the following terms have the meaning indicated below.

In some embodiments, the compounds disclosed herein contain one or more asymmetric centers and thus give rise to enantiomers, diastereomers, and other stereoisomeric forms that are defined, in terms of absolute stereochemistry, as (*R*)- or (*S*)-. Unless stated otherwise, it is intended that all stereoisomeric forms of the compounds disclosed herein are contemplated by this disclosure. When the compounds described herein contain alkene double bonds, and unless specified otherwise, it is intended that this disclosure includes both *E* and *Z* geometric isomers (*e.g., cis* or *trans*.) Likewise, all possible isomers, as well as their racemic and optically pure forms, and all tautomeric forms are also intended to be included. The term "geometric isomer" refers to *E* or *Z* geometric isomers (*e.g., cis* or *trans*) of an alkene double bond. The term "positional isomer" refers to structural isomers around a central ring, such as *ortho-, meta-,* and *para-* isomers around a benzene ring.

A "tautomer" refers to a molecule wherein a proton shift from one atom of a molecule to another atom of the same molecule is possible. In certain embodiments, the compounds presented herein exist as tautomers. In circumstances where tautomerization is possible, a chemical equilibrium of the tautomers will exist. The exact ratio of the tautomers depends on several factors, including physical state, temperature, solvent, and pH. Some examples of tautomeric equilibrium include:

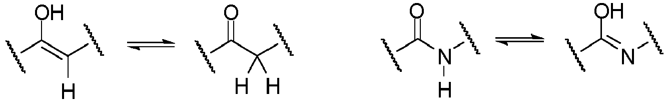

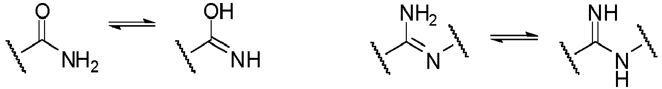

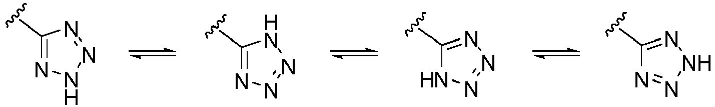

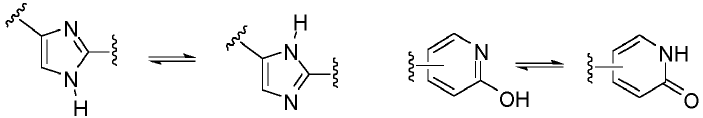

"Pharmaceutically acceptable salt" includes both acid and base addition salts. A pharmaceutically acceptable salt of any one of the compounds described herein is intended to encompass any and all pharmaceutically suitable salt forms. Preferred pharmaceutically acceptable salts of the compounds described herein are pharmaceutically acceptable acid addition salts, and pharmaceutically acceptable base addition salts.

"Pharmaceutically acceptable acid addition salt" refers to those salts which retain the biological effectiveness and properties of the free bases, which are not biologically or otherwise undesirable, and which are formed with inorganic acids such as hydrochloric acid, hydrobromic acid, sulfuric acid, nitric acid, phosphoric acid, hydroiodic acid, hydrofluoric acid, phosphorous acid, and the like. Also included are salts that are formed with organic acids such as aliphatic mono- and dicarboxylic acids, phenyl-substituted alkanoic acids, hydroxy alkanoic acids, alkanedioic acids, aromatic acids, aliphatic and aromatic sulfonic acids, etc. and include, for example, acetic acid, trifluoroacetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, *p*-toluenesulfonic acid, salicylic acid, and the like. Exemplary salts thus include sulfates, pyrosulfates, bisulfates, sulfites, bisulfites, nitrates, phosphates, monohydrogenphosphates, dihydrogenphosphates, metaphosphates, pyrophosphates, chlorides, bromides, iodides, acetates, trifluoroacetates, propionates, caprylates, isobutyrates, oxalates, malonates, succinate suberates, sebacates, fumarates, maleates, mandelates, benzoates, chlorobenzoates, methylbenzoates, dinitrobenzoates, phthalates, benzenesulfonates, toluenesulfonates, phenylacetates, citrates, lactates, malates, tartrates, methanesulfonates, and the like. Also contemplated are salts of amino acids, such as arginates, gluconates, and galacturonates (see, for example, Berge S.M. et al., "Pharmaceutical Salts," Journal of Pharmaceutical Science, 66:1-19 (1997)). Acid addition salts of basic compounds are prepared by contacting the free base forms with a sufficient amount of the desired acid to produce the salt.

"Pharmaceutically acceptable base addition salt" refers to those salts that retain the biological effectiveness and properties of the free acids, which are not biologically or otherwise undesirable. These salts are prepared from addition of an inorganic base or an organic base to the free acid. In some embodiments, pharmaceutically acceptable base addition salts are formed with metals or amines, such as alkali and alkaline earth metals or organic amines. Salts derived from inorganic bases include, but are not limited to, sodium, potassium, lithium, ammonium, calcium, magnesium, iron, zinc, copper, manganese, aluminum salts, and the like. Salts derived from organic bases include, but are not limited to, salts of primary, secondary, and tertiary amines, substituted amines including naturally occurring substituted amines, cyclic amines and basic ion exchange resins, for example, isopropylamine, trimethylamine, diethylamine, triethylamine, tripropylamine, ethanolamine, diethanolamine, 2-dimethylaminoethanol, 2-diethylaminoethanol, dicyclohexylamine, lysine, arginine, histidine, caffeine, procaine, *N*,*N-*dibenzylethylenediamine, chloroprocaine, hydrabamine, choline, betaine, ethylenediamine, ethylenedianiline, *N-*methylglucamine, glucosamine, methylglucamine, theobromine, purines, piperazine, piperidine, *N*-ethylpiperidine, polyamine resins, and the like. See Berge et al., *supra.*

As used herein, "treatment" or "treating " or "palliating" or "ameliorating" are used interchangeably herein. These terms refer to an approach for obtaining beneficial or desired results including but not limited to therapeutic benefit and/or a prophylactic benefit. By "therapeutic benefit" is meant eradication or amelioration of the underlying disorder being treated. Also, a therapeutic benefit is achieved with the eradication or amelioration of one or more of the physiological symptoms associated with the underlying disorder such that an improvement is observed in the patient, notwithstanding that the patient is still afflicted with the underlying disorder. For prophylactic benefit, the compositions are administered to a patient at risk of developing a particular disease, or to a patient reporting one or more of the physiological symptoms of a disease, even though a diagnosis of this disease has not been made.

The reference to methods of treatment by therapy or surgery or in vivo diagnosis of this description are to be interpreted as reference to compounds, pharmaceutical compositions and medicaments of the present inventions for use in those methods.

### Compounds

The compounds described herein are modulators of ABHD12. The compounds described herein, and compositions comprising these compounds, are useful for the treatment of a disease or disorder selected from a neuropsychiatric disorder, an autoimmune disease, a neuroinflammatory disease, a neurodegenerative disease, and cancer. In some embodiments compounds described herein, and compositions comprising these compounds, stimulate a patient's immune system to treat a disease. In some embodiments, the disease is an infectious disease. In some embodiments, the disease is cancer. **Preparation of Compounds**

The compounds used in the reactions described herein are made according to organic synthesis techniques, starting from commercially available chemicals and/or from compounds described in the chemical literature. "Commercially available chemicals" are obtained from standard commercial sources including Acros Organics (Geel, Belgium), Aldrich Chemical (Milwaukee, WI, including Sigma Chemical and Fluka), Apin Chemicals Ltd. (Milton Park, UK), Ark Pharm, Inc. (Libertyville, IL), Avocado Research (Lancashire, U.K.), BDH Inc. (Toronto, Canada), Bionet (Cornwall, U.K.), Chemservice Inc. (West Chester, PA), Combi-blocks (San Diego, CA), Crescent Chemical Co. (Hauppauge, NY), eMolecules (San Diego, CA), Fisher Scientific Co. (Pittsburgh, PA), Fisons Chemicals (Leicestershire, UK), Frontier Scientific (Logan, UT), ICN Biomedicals, Inc. (Costa Mesa, CA), Key Organics (Cornwall, U.K.), Lancaster Synthesis (Windham, NH), Matrix Scientific, (Columbia, SC), Maybridge Chemical Co. Ltd. (Cornwall, U.K.), Parish Chemical Co. (Orem, UT), Pfaltz & Bauer, Inc. (Waterbury, CN), Polyorganix (Houston, TX), Pierce Chemical Co. (Rockford, IL), Riedel de Haen AG (Hanover, Germany), Ryan Scientific, Inc. (Mount Pleasant, SC), Spectrum Chemicals (Gardena, CA), Sundia Meditech, (Shanghai, China), TCI America (Portland, OR), Trans World Chemicals, Inc. (Rockville, MD), and WuXi (Shanghai, China).

Suitable reference books and treatises that detail the synthesis of reactants useful in the preparation of compounds described herein, or provide references to articles that describe the preparation, include for example, "Synthetic Organic Chemistry", John Wiley & Sons, Inc., New York; S. R. Sandler et al., "Organic Functional Group Preparations," 2nd Ed., Academic Press, New York, 1983; H. O. House, "Modern Synthetic Reactions", 2nd Ed., W. A. Benjamin, Inc. Menlo Park, Calif. 1972; T. L. Gilchrist, "Heterocyclic Chemistry", 2nd Ed., John Wiley & Sons, New York, 1992; J. March, "Advanced Organic Chemistry: Reactions, Mechanisms and Structure", 4th Ed., Wiley-Interscience, New York, 1992. Additional suitable reference books and treatises that detail the synthesis of reactants useful in the preparation of compounds described herein, or provide references to articles that describe the preparation, include for example, Fuhrhop, J. and Penzlin G. "Organic Synthesis: Concepts, Methods, Starting Materials", Second, Revised and Enlarged Edition (1994) John Wiley & Sons ISBN: 3-527-29074-5; Hoffman, R.V. "Organic Chemistry, An Intermediate Text" (1996) Oxford University Press, ISBN 0-19-509618-5; Larock, R. C. "Comprehensive Organic Transformations: A Guide to Functional Group Preparations" 2nd Edition (1999) Wiley-VCH, ISBN: 0-471-19031-4; March, J. "Advanced Organic Chemistry: Reactions, Mechanisms, and Structure" 4th Edition (1992) John Wiley & Sons, ISBN: 0-471-60180-2; Otera, J. (editor) "Modern Carbonyl Chemistry" (2000) Wiley-VCH, ISBN: 3-527-29871-1; Patai, S. "Patai's 1992 Guide to the Chemistry of Functional Groups" (1992) Interscience ISBN: 0-471-93022-9; Solomons, T. W. G. "Organic Chemistry" 7th Edition (2000) John Wiley & Sons, ISBN: 0-471-19095-0; Stowell, J.C., "Intermediate Organic Chemistry" 2nd Edition (1993) Wiley-Interscience, ISBN: 0-471-57456-2; "Industrial Organic Chemicals: Starting Materials and Intermediates: An Ullmann's Encyclopedia" (1999) John Wiley & Sons, ISBN: 3-527-29645-X, in 8 volumes; "Organic Reactions" (1942-2000) John Wiley & Sons, in over 55 volumes; and "Chemistry of Functional Groups" John Wiley & Sons, in 73 volumes.

Specific and analogous reactants are also identified through the indices of known chemicals prepared by the Chemical Abstract Service of the American Chemical Society, which are available in most public and university libraries, as well as through on-line databases (the American Chemical Society, Washington, D.C.). Chemicals that are known but not commercially available in catalogs are optionally prepared by custom chemical synthesis houses, where many of the standard chemical supply houses (*e*.*g*., those listed above) provide custom synthesis services. A reference for the preparation and selection of pharmaceutical salts of the compounds described herein is P. H. Stahl & C. G. Wermuth "Handbook of Pharmaceutical Salts", Verlag Helvetica Chimica Acta, Zurich, 2002.

### Further Forms of Compounds Disclosed Herein

### Isomers

Furthermore, in some embodiments, the compounds described herein exist as geometric isomers. In some embodiments, the compounds described herein possess one or more double bonds. The compounds presented herein include all cis, trans, syn, anti, entgegen (*E*), and zusammen (Z) isomers as well as the corresponding mixtures thereof. In some situations, compounds exist as tautomers. The compounds described herein include all possible tautomers within the formulas described herein. In some situations, the compounds described herein possess one or more chiral centers and each center exists in the R configuration or S configuration. The compounds described herein include all diastereomeric, enantiomeric, and epimeric forms as well as the corresponding mixtures thereof. In additional embodiments of the compounds and methods provided herein, mixtures of enantiomers and/or diastereoisomers, resulting from a single preparative step, combination, or interconversion, are useful for the applications described herein. In some embodiments, the compounds described herein are prepared as optically pure enantiomers by chiral chromatographic resolution of the racemic mixture. In some embodiments, the compounds described herein are prepared as their individual stereoisomers by reacting a racemic mixture of the compound with an optically active resolving agent to form a pair of diastereoisomeric compounds, separating the diastereomers, and recovering the optically pure enantiomers. In some embodiments, dissociable complexes are preferred (e.g., crystalline diastereomeric salts). In some embodiments, the diastereomers have distinct physical properties (e.g., melting points, boiling points, solubilities, reactivity, etc.) and are separated by taking advantage of these dissimilarities. In some embodiments, the diastereomers are separated by chiral chromatography, or preferably, by separation/resolution techniques based upon differences in solubility. In some embodiments, the optically pure enantiomer is then recovered, along with the resolving agent, by any practical means that does not result in racemization.

### Labeled compounds

In some embodiments, the compounds described herein exist in their isotopically-labeled forms. In some embodiments, the methods disclosed herein include methods of treating diseases by administering such isotopically-labeled compounds. In some embodiments, the methods disclosed herein include methods of treating diseases by administering such isotopically-labeled compounds as pharmaceutical compositions. Thus, in some embodiments, the compounds disclosed herein include isotopically-labeled compounds, which are identical to those recited herein, but for the fact that one or more atoms are replaced by an atom having an atomic mass or mass number different from the atomic mass or mass number usually found in nature. Examples of isotopes that are incorporated into compounds described herein include isotopes of hydrogen, carbon, nitrogen, oxygen, phosphorous, sulfur, fluorine, and chloride, such as ²H, ³H, ¹³C, ¹⁴C, ¹⁵N, ¹⁸O, ¹⁷O, ³¹P, ³²P, ³⁵S, ¹⁸F, and ³⁶Cl, respectively. Compounds described herein, and pharmaceutically acceptable salts, esters, solvate, hydrates, or derivatives thereof which contain the aforementioned isotopes and/or other isotopes of other atoms are within the scope of this invention. Certain isotopically-labeled compounds, for example those into which radioactive isotopes such as ³H and ¹⁴C are incorporated, are useful in drug and/or substrate tissue distribution assays. Tritiated, i. e., ³H and carbon-14, i. e., ¹⁴C, isotopes are particularly preferred for their ease of preparation and detectability. Further, substitution with heavy isotopes such as deuterium, i.e., ²H, produces certain therapeutic advantages resulting from greater metabolic stability, for example increased *in vivo* half-life or reduced dosage requirements. In some embodiments, the isotopically labeled compounds, pharmaceutically acceptable salt, ester, solvate, hydrate, or derivative thereof is prepared by any suitable method.

In some embodiments, the compounds described herein are labeled by other means, including, but not limited to, the use of chromophores or fluorescent moieties, bioluminescent labels, or chemiluminescent labels.

### Pharmaceutically acceptable salts

In some embodiments, the compounds described herein exist as their pharmaceutically acceptable salts. In some embodiments, the methods disclosed herein include methods of treating diseases by administering such pharmaceutically acceptable salts. In some embodiments, the methods disclosed herein include methods of treating diseases by administering such pharmaceutically acceptable salts as pharmaceutical compositions.

In some embodiments, the compounds described herein possess acidic or basic groups and therefore react with any of a number of inorganic or organic bases, and inorganic and organic acids, to form a pharmaceutically acceptable salt. In some embodiments, these salts are prepared *in situ* during the final isolation and purification of the compounds described herein, or by separately reacting a purified compound in its free form with a suitable acid or base, and isolating the salt thus formed.

### Solvates

In some embodiments, the compounds described herein exist as solvates. In some embodiments are methods of treating diseases by administering such solvates. Further described herein are methods of treating diseases by administering such solvates as pharmaceutical compositions.

Solvates contain either stoichiometric or non-stoichiometric amounts of a solvent, and, in some embodiments, are formed during the process of crystallization with pharmaceutically acceptable solvents such as water, ethanol, and the like. Hydrates are formed when the solvent is water, or alcoholates are formed when the solvent is alcohol. Solvates of the compounds described herein are conveniently prepared or formed during the processes described herein. By way of example only, hydrates of the compounds described herein are conveniently prepared by recrystallization from an aqueous/organic solvent mixture, using organic solvents including, but not limited to, dioxane, tetrahydrofuran, or MeOH. In addition, the compounds provided herein exist in unsolvated as well as solvated forms. In general, the solvated forms are considered equivalent to the unsolvated forms for the purposes of the compounds and methods provided herein.

### Pharmaceutical Compositions

In certain embodiments, the compounds described herein are administered as a pure chemical. In some embodiments, a compound described herein is combined with a pharmaceutically suitable or acceptable carrier (also referred to herein as a pharmaceutically suitable (or acceptable) excipient, physiologically suitable (or acceptable) excipient, or physiologically suitable (or acceptable) carrier) selected on the basis of a chosen route of administration and standard pharmaceutical practice as described, for example, in Remington: The Science and Practice of Pharmacy (Gennaro, 21st Ed. Mack Pub. Co., Easton, PA (2005)).

Accordingly, provided herein is a pharmaceutical composition comprising at least one compound described herein, or a stereoisomer, pharmaceutically acceptable salt, hydrate, solvate, or N-oxide thereof, together with one or more pharmaceutically acceptable carriers. The carrier(s) (or excipient(s)) is acceptable or suitable if the carrier is compatible with the other ingredients of the composition and not deleterious to the recipient (*i*.*e*., the subject) of the composition.

One embodiment provides a pharmaceutical composition comprising a pharmaceutically acceptable excipient and a compound described herein, or a pharmaceutically acceptable salt thereof.

Another embodiment provides a pharmaceutical composition consisting essentially of a pharmaceutically acceptable excipient and a compound described herein, or a pharmaceutically acceptable salt thereof.

In certain embodiments, the compound described herein is substantially pure, in that it contains less than about 5%, or less than about 1%, or less than about 0.1%, of other organic small molecules, such as contaminating intermediates or by-products that are created, for example, in one or more of the steps of a synthesis method.

These formulations include those suitable for oral, rectal, topical, buccal, parenteral (*e*.*g*., subcutaneous, intramuscular, intradermal, or intravenous), vaginal, ophthalmic, or aerosol administration, although the most suitable form of administration in any given case will depend on the degree and severity of the condition being treated and on the nature of the particular compound being used. For example, disclosed compositions are formulated as a unit dose, and/or are formulated for oral or subcutaneous administration.

Exemplary pharmaceutical compositions are used in the form of a pharmaceutical preparation, for example, in solid, semisolid, or liquid form, which includes one or more of a disclosed compound, as an active ingredient, in a mixture with an organic or inorganic carrier or excipient suitable for external, enteral, or parenteral applications. In some embodiments, the active ingredient is compounded, for example, with the usual non-toxic, pharmaceutically acceptable carriers for tablets, pellets, capsules, suppositories, solutions, emulsions, suspensions, and any other form suitable for use. The active object compound is included in the pharmaceutical composition in an amount sufficient to produce the desired effect upon the process or condition of the disease.

In some embodiments for preparing solid compositions such as tablets, the principal active ingredient is mixed with a pharmaceutical carrier, e.g., conventional tableting ingredients such as corn starch, lactose, sucrose, sorbitol, talc, stearic acid, magnesium stearate, dicalcium phosphate, or gums, and other pharmaceutical diluents, e.g., water, to form a solid preformulation composition containing a homogeneous mixture of a disclosed compound or a non-toxic pharmaceutically acceptable salt thereof. When referring to these preformulation compositions as homogeneous, it is meant that the active ingredient is dispersed evenly throughout the composition so that the composition is readily subdivided into equally effective unit dosage forms such as tablets, pills, and capsules.

In solid dosage forms for oral administration (capsules, tablets, pills, dragees, powders, granules and the like), the subject composition is mixed with one or more pharmaceutically acceptable carriers, such as sodium citrate or dicalcium phosphate, and/or any of the following: (1) fillers or extenders, such as starches, cellulose, microcrystalline cellulose, silicified microcrystalline cellulose, lactose, sucrose, glucose, mannitol, and/or silicic acid; (2) binders, such as, for example, carboxymethylcellulose, hypromellose, alginates, gelatin, polyvinyl pyrrolidone, sucrose and/or acacia; (3) humectants, such as glycerol; (4) disintegrating agents, such as crospovidone, croscarmellose sodium, sodium starch glycolate, agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, docusate sodium, cetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, in some embodiments, the compositions comprise buffering agents. In some embodiments, solid compositions of a similar type are also employed as fillers in soft and hard-filled gelatin capsules using such excipients as lactose or milk sugars, as well as high molecular weight polyethylene glycols and the like.

In some embodiments, a tablet is made by compression or molding, optionally with one or more accessory ingredients. In some embodiments, compressed tablets are prepared using binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. In some embodiments, molded tablets are made by molding in a suitable machine a mixture of the subject composition moistened with an inert liquid diluent. In some embodiments, tablets, and other solid dosage forms, such as dragees, capsules, pills, and granules, are scored or prepared with coatings and shells, such as enteric coatings and other coatings.

Compositions for inhalation or insufflation include solutions and suspensions in pharmaceutically acceptable aqueous or organic solvents, or mixtures thereof, and powders. Liquid dosage forms for oral administration include pharmaceutically acceptable emulsions, microemulsions, solutions, suspensions, syrups, and elixirs. In addition to the subject composition, in some embodiments, the liquid dosage forms contain inert diluents, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (optionally, cottonseed, groundnut, corn, germ, olive, castor and sesame oils), glycerol, tetrahydrofuryl alcohol, polyethylene glycols and fatty acid esters of sorbitan, cyclodextrins, and mixtures thereof.

In some embodiments, suspensions, in addition to the subject composition, contain suspending agents as, for example, ethoxylated isostearyl alcohols, polyoxyethylene sorbitol and sorbitan esters, microcrystalline cellulose, aluminum metahydroxide, bentonite, agar-agar, tragacanth, and mixtures thereof.

In some embodiments, formulations for rectal or vaginal administration are presented as a suppository, which are prepared by mixing a subject composition with one or more suitable non-irritating excipients or carriers comprising, for example, cocoa butter, polyethylene glycol, a suppository wax or a salicylate, and which is solid at room temperature, but liquid at body temperature and, therefore, will melt in the body cavity and release the active agent.

Dosage forms for transdermal administration of a subject composition include powders, sprays, ointments, pastes, creams, lotions, gels, solutions, patches, and inhalants. In some embodiments, the active component is mixed under sterile conditions with a pharmaceutically acceptable carrier, and with any preservatives, buffers, or propellants as required.

In some embodiments, the ointments, pastes, creams, and gels contain, in addition to a subject composition, excipients, such as animal and vegetable fats, oils, waxes, paraffins, starch, tragacanth, cellulose derivatives, polyethylene glycols, silicones, bentonites, silicic acid, talc, and zinc oxide, or mixtures thereof.

In some embodiments, powders and sprays contain, in addition to a subject composition, excipients such as lactose, talc, silicic acid, aluminum hydroxide, calcium silicates and polyamide powder, or mixtures of these substances. In some embodiments, sprays additionally contain customary propellants, such as chlorofluorohydrocarbons and volatile unsubstituted hydrocarbons, such as butane and propane.

In some embodiments, the compounds described herein are formulated as eye drops for ophthalmic administration.

Compositions and compounds disclosed herein alternatively are administered by aerosol. This is accomplished by preparing an aqueous aerosol, liposomal preparation, or solid particles containing the compound. In some embodiments, a non-aqueous (e.g., fluorocarbon propellant) suspension is used. In some embodiments, sonic nebulizers are used because they minimize exposing the agent to shear, which results in degradation of the compounds contained in the subject compositions. Ordinarily, an aqueous aerosol is made by formulating an aqueous solution or suspension of a subject composition together with conventional pharmaceutically acceptable carriers and stabilizers. The carriers and stabilizers vary with the requirements of the particular subject composition, but typically include non-ionic surfactants (Tweens, Pluronics, or polyethylene glycol), innocuous proteins like serum albumin, sorbitan esters, oleic acid, lecithin, and amino acids such as glycine, buffers, salts, sugars, or sugar alcohols. Aerosols generally are prepared from isotonic solutions.

Pharmaceutical compositions suitable for parenteral administration comprise a subject composition in combination with one or more pharmaceutically-acceptable sterile isotonic aqueous or non-aqueous solutions, dispersions, suspensions or emulsions, or sterile powders which are reconstituted into sterile injectable solutions or dispersions just prior to use, which, in some embodiments, contain antioxidants, buffers, bacteriostats, solutes which render the formulation isotonic with the blood of the intended recipient or suspending or thickening agents.

Examples of suitable aqueous and non-aqueous carriers which are employed in the pharmaceutical compositions include water, ethanol, polyols (such as glycerol, propylene glycol, polyethylene glycol, and the like), and suitable mixtures thereof, vegetable oils such as olive oil, and injectable organic esters such as ethyl oleate and cyclodextrins. Proper fluidity is maintained, for example, by the use of coating materials, such as lecithin, by the maintenance of the required particle size in the case of dispersions, and by the use of surfactants.

Also contemplated are enteral pharmaceutical formulations including a disclosed compound, an enteric material, and a pharmaceutically acceptable carrier or excipient thereof. Enteric materials refer to polymers that are substantially insoluble in the acidic environment of the stomach, and that are predominantly soluble in intestinal fluids at specific pHs. The small intestine is the part of the gastrointestinal tract (gut) between the stomach and the large intestine, and includes the duodenum, jejunum, and ileum. The pH of the duodenum is about 5.5, the pH of the jejunum is about 6.5, and the pH of the distal ileum is about 7.5. Accordingly, enteric materials are not soluble, for example, until a pH of about 5.0, of about 5.2, of about 5.4, of about 5.6, of about 5.8, of about 6.0, of about 6.2, of about 6.4, of about 6.6, of about 6.8, of about 7.0, of about 7.2, of about 7.4, of about 7.6, of about 7.8, of about 8.0, of about 8.2, of about 8.4, of about 8.6, of about 8.8, of about 9.0, of about 9.2, of about 9.4, of about 9.6, of about 9.8, or of about 10.0. Exemplary enteric materials include cellulose acetate phthalate (CAP), hydroxypropyl methylcellulose phthalate (HPMCP), polyvinyl acetate phthalate (PVAP), hydroxypropyl methylcellulose acetate succinate (HPMCAS), cellulose acetate trimellitate, hydroxypropyl methylcellulose succinate, cellulose acetate succinate, cellulose acetate hexahydrophthalate, cellulose propionate phthalate, cellulose acetate maleate, cellulose acetate butyrate, cellulose acetate propionate, copolymer of methylmethacrylic acid and methyl methacrylate, copolymer of methyl acrylate, methylmethacrylate and methacrylic acid, copolymer of methylvinyl ether and maleic anhydride (Gantrez ES series), ethyl methyacrylate-methylmethacrylate-chlorotrimethylammonium ethyl acrylate copolymer, natural resins such as zein, shellac and copal collophorium, and several commercially available enteric dispersion systems (*e*.*g*., Eudragit L30D55, Eudragit FS30D, Eudragit L100, Eudragit S100, Kollicoat EMM30D, Estacryl 30D, Coateric, and Aquateric). The solubility of each of the above materials is either known or is readily determinable *in vitro.*

The dose of the composition comprising at least one compound described herein differs, depending upon the patient's (e.g., human) condition, that is, stage of the disease, general health status, age, and other factors.

Pharmaceutical compositions are administered in a manner appropriate to the disease to be treated (or prevented). An appropriate dose and a suitable duration and frequency of administration will be determined by such factors as the condition of the patient, the type and severity of the patient's disease, the particular form of the active ingredient, and the method of administration. In general, an appropriate dose and treatment regimen provides the composition(s) in an amount sufficient to provide therapeutic and/or prophylactic benefit (e.g., an improved clinical outcome, such as more frequent complete or partial remissions, or longer disease-free and/or overall survival, or a lessening of symptom severity. Optimal doses are generally determined using experimental models and/or clinical trials. In some embodiments, the optimal dose depends upon the body mass, weight, or blood volume of the patient.

Oral doses typically range from about 1.0 mg to about 1000 mg, one to four times, or more, per day.

### Methods

Disclosed herein are methods of modulating the activity of ABHD12. Contemplated methods, for example, comprise exposing said enzyme to a compound described herein. In some embodiments, the compound utilized by one or more of the foregoing methods is one of the generic, subgeneric, or specific compounds described herein, such as a compound described herein. In some embodiments, provided herein is a compound described herein wherein the compound is an ABHD12 inhibitor. In some embodiments, provided herein is a compound described herein wherein the compound is a selective ABHD12 inhibitor. The ability of compounds described herein to modulate or inhibit ABHD12 is evaluated by procedures known in the art and/or described herein. Another aspect of this disclosure provides methods of treating a disease associated with expression or activity of ABHD12 in a patient. In some embodiments, provided herein is a compound described herein wherein the compound is selective in inhibiting ABHD12 as compared to inhibition of other serine hydrolases. In some embodiments, provided herein is a compound described herein wherein the compound is 10, 100, or 1000 fold selective in inhibiting ABHD12 as compared to inhibition of other serine hydrolases.

In another embodiment is a method of treating a disease or disorder in a patient in need thereof, comprising administering to the patient a therapeutically effective amount of a compound described herein, or a solvate, hydrate, tautomer, N-oxide, or a pharmaceutically acceptable salt thereof, wherein the disease or disorder is a neuropsychiatric disorder, an autoimmune disease, a neuroinflammatory disease, a neurodegenerative disease, or cancer. In another embodiment is a method of treating a neuropsychiatric disorder in a patient in need thereof, comprising administering to the patient a therapeutically effective amount of a compound described herein, or a solvate, hydrate, tautomer, N-oxide, or a pharmaceutically acceptable salt thereof. In another embodiment is a method of treating an autoimmune disease in a patient in need thereof, comprising administering to the patient a therapeutically effective amount of a compound described herein, or a solvate, hydrate, tautomer, *N*-oxide, or a pharmaceutically acceptable salt thereof. In another embodiment is a method of treating a neuroinflammatory disease in a patient in need thereof, comprising administering to the patient a therapeutically effective amount of a compound described herein, or a solvate, hydrate, tautomer, *N*-oxide, or a pharmaceutically acceptable salt thereof. In another embodiment is a method of treating a neurodegenerative disease in a patient in need thereof, comprising administering to the patient a therapeutically effective amount of a compound described herein, or a solvate, hydrate, tautomer, *N*-oxide, or a pharmaceutically acceptable salt thereof. In another embodiment is a method of treating cancer in a patient in need thereof, comprising administering to the patient a therapeutically effective amount of a compound described herein, or a solvate, hydrate, tautomer, *N*-oxide, or a pharmaceutically acceptable salt thereof.

In another embodiment is a method of treating cancer in a patient in need thereof, comprising administering to the patient a therapeutically effective amount of a compound described herein, or a solvate, *N*-oxide, stereoisomer, or pharmaceutically acceptable salt thereof, wherein the compound is an immunotherapeutic agent.

In some embodiments, a compound described herein stimulates a patient's immune system to treat a disease. In some embodiments is a method of treating a disease in a patient in need thereof, comprising administering to the patient a therapeutically effective amount of a compound described herein, or a solvate, *N*-oxide, stereoisomer, or pharmaceutically acceptable salt thereof, wherein administration of the compound stimulates the patient's immune system. In another embodiment is a method of treating cancer in a patient in need thereof, comprising administering to the patient a therapeutically effective amount of a compound described herein, or a solvate, N-oxide, stereoisomer, or pharmaceutically acceptable salt thereof, wherein administration of the compound stimulates the patient's immune system. In another embodiment is a method of treating cancer in a patient in need thereof, comprising administering to the patient a therapeutically effective amount of a compound described herein, or a solvate, *N-*oxide, stereoisomer, or pharmaceutically acceptable salt thereof, wherein administration of the compound initiates an immune response.

In another embodiment is a method of treating an infectious disease in a patient in need thereof, comprising administering to the patient a therapeutically effective amount of a compound described herein, or a solvate, *N*-oxide, stereoisomer, or pharmaceutically acceptable salt thereof, wherein the compound is an immunotherapeutic agent.

In another embodiment is a method of treating an infectious disease in a patient in need thereof, comprising administering to the patient a therapeutically effective amount of a compound described herein, or a solvate, *N*-oxide, stereoisomer, or pharmaceutically acceptable salt thereof, wherein administration of the compound stimulates the patient's immune system. In another embodiment is a method of treating an infectious disease in a patient in need thereof, comprising administering to the patient a therapeutically effective amount of a compound described herein, or a solvate, *N*-oxide, stereoisomer, or pharmaceutically acceptable salt thereof, wherein administration of the compound stimulates the patient's immune system. In another embodiment is a method of treating an infectious disease in a patient in need thereof, comprising administering to the patient a therapeutically effective amount of a compound described herein, or a solvate, *N*-oxide, stereoisomer, or pharmaceutically acceptable salt thereof, wherein administration of the compound initiates an immune response.

In certain embodiments, a disclosed compound utilized by one or more of the foregoing methods is one of the specific compounds described herein.

Disclosed compounds are administered to patients (animals and humans) in need of such treatment in dosages that will provide optimal pharmaceutical efficacy. It will be appreciated that the dose required for use in any particular application will vary from patient to patient, not only with the particular compound or composition selected, but also with the route of administration, the nature of the condition being treated, the age and condition of the patient, concurrent medication or special diets then being followed by the patient, and other factors, with the appropriate dosage ultimately being at the discretion of the attendant physician. For treating clinical conditions and diseases noted above, a contemplated compound disclosed herein is administered orally, subcutaneously, topically, parenterally, by inhalation spray, or rectally in dosage unit formulations containing conventional non-toxic pharmaceutically acceptable carriers, adjuvants and vehicles. Parenteral administration includes subcutaneous injections, intravenous, or intramuscular injections or infusion techniques.

Also contemplated herein are combination therapies, for example, co-administering a disclosed compound and an additional active agent, as part of a specific treatment regimen intended to provide the beneficial effect from the co-action of these therapeutic agents. The beneficial effect of the combination includes, but is not limited to, pharmacokinetic or pharmacodynamic co-action resulting from the combination of therapeutic agents. Administration of these therapeutic agents in combination typically is carried out over a defined time period (usually weeks, months, or years depending upon the combination selected). Combination therapy is intended to embrace administration of multiple therapeutic agents in a sequential manner, that is, wherein each therapeutic agent is administered at a different time, as well as administration of these therapeutic agents, or at least two of the therapeutic agents, in a substantially simultaneous manner.

Substantially simultaneous administration is accomplished, for example, by administering to the subject a single formulation or composition, (e.g., a tablet or capsule having a fixed ratio of each therapeutic agent or in multiple, single formulations (e.g., capsules) for each of the therapeutic agents. Sequential or substantially simultaneous administration of each therapeutic agent is effected by any appropriate route including, but not limited to, oral routes, intravenous routes, intramuscular routes, and direct absorption through mucous membrane tissues. The therapeutic agents are administered by the same route or by different routes. For example, a first therapeutic agent of the combination selected is administered by intravenous injection while the other therapeutic agents of the combination are administered orally. Alternatively, for example, all therapeutic agents are administered orally or all therapeutic agents are administered by intravenous injection.

Combination therapy also embraces the administration of the therapeutic agents as described above in further combination with other biologically active ingredients and non-drug therapies. Where the combination therapy further comprises a non-drug treatment, the non-drug treatment is conducted at any suitable time so long as a beneficial effect from the co-action of the combination of the therapeutic agents and non-drug treatment is achieved. For example, in appropriate cases, the beneficial effect is still achieved when the non-drug treatment is temporally removed from the administration of the therapeutic agents, perhaps by days or even weeks.

The components of the combination are administered to a patient simultaneously or sequentially. It will be appreciated that the components are present in the same pharmaceutically acceptable carrier and, therefore, are administered simultaneously. Alternatively, the active ingredients are present in separate pharmaceutical carriers, such as, conventional oral dosage forms, that are administered either simultaneously or sequentially.

The following examples are provided merely as illustrative of various embodiments and shall not be construed to limit the invention in any way.

### EXAMPLES

### List of abbreviations

As used above, and throughout the description of the invention, the following abbreviations, unless otherwise indicated, shall be understood to have the following meanings:
- ACN or MeCN: acetonitrile
- Bn: benzyl
- BOC or Boc: *tert*-butyl carbamate
- CDI: 1,1'-carbonyldiimidazole
- Cy: cyclohexyl
- DCE: dichloroethane (ClCH₂CH₂Cl)
- DCM: dichloromethane (CH₂Cl₂)
- DIPEA or DIEA: diisopropylethylamine
- DMAP: 4-(*N*,*N-*dimethylamino)pyridine
- DMF: dimethylformamide
- DMA: *N,N*-dimethylacetamide
- DMSO: dimethylsulfoxide
- equiv: equivalent(s)
- Et: ethyl
- EtOH: ethanol
- EtOAc: ethyl acetate
- HATU: 1-[bis(dimethylamino)methylene]-1H-1,2,3-triazolo[4,5-b]pyridinium 3-oxid hexafluorophosphate
- HPLC: high performance liquid chromatography
- LAH: lithium aluminum hydride
- Me: methyl
- MeOH: methanol
- MS: mass spectroscopy
- NMM: *N*-methylmorpholine
- NMR: nuclear magnetic resonance
- PMB: *para*-methoxybenzyl
- TEA: triethylamine
- TFA: trifluoroacetic acid
- THF: tetrahydrofuran
- TLC: thin layer chromatography

### I. Chemical Synthesis

Unless otherwise noted, reagents and solvents were used as received from commercial suppliers. Anhydrous solvents and oven-dried glassware were used for synthetic transformations sensitive to moisture and/or oxygen. Yields were not optimized. Reaction times are approximate and were not optimized. Column chromatography and thin layer chromatography (TLC) were performed on silica gel unless otherwise noted. Spectra are given in ppm (δ) and coupling constants (*J*) are reported in Hertz. For proton spectra the solvent peak was used as the reference peak.

### Example 1: 1-(Pyridin-3-yl)-3-(1-(6-(trifluoromethyl)pyridin-2-yl)piperidin-4-yl)urea

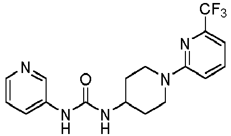

### Step 1: Preparation of t-butyl (1-(6-(trifluoromethyl)pyridin-2-yl)piperidin-4-yl)carbamate

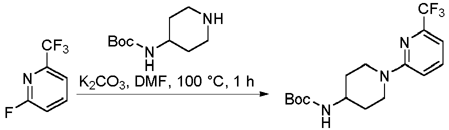

A mixture of 2-fluoro-6-(trifluoromethyl)pyridine (82 mg, 0.50 mmol, 1.00 equiv) and t-butyl N-(piperidin-4-yl)carbamate (100 mg, 0.50 mmol, 1.00 equiv) and K₂CO₃ (138 mg, 1.00 mmol, 2.00 equiv) in DMF (3 mL) was stirred for 1 h at 100 °C. The reaction was quenched with water (20 mL) and extracted with EtOAc (3 x 20 mL). The organic layers were combined, washed with brine (20 mL), dried over anhydrous sodium sulfate, filtered and concentrated under reduced pressure to provide t-butyl (1-(6-(trifluoromethyl)pyridin-2-yl)piperidin-4-yl)carbamate (155 mg, 90%) as a yellow solid. LCMS (ESI, *m*/*z):* 346 [M+H]⁺.

### Step 2: Preparation of 1-(6-(trifluoromethyl)pyridin-2-yl)piperidin-4-amine

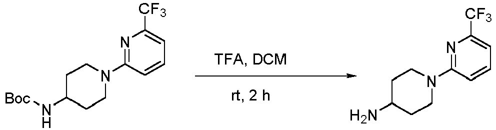

A solution of *t*-butyl (1-(6-(trifluoromethyl)pyridin-2-yl)piperidin-4-yl)carbamate (155 mg, 0.50 mmol, 1.00 equiv) in TFA (1 mL) and DCM (5 mL) was stirred for 2 h at rt. The resulting mixture was concentrated under reduced pressure. The residue was neutralized to pH 9 with NH_{3•}H₂O. The resulting mixture was concentrated under reduced pressure. The residue was purified by flash chromatography to provide 1-(6-(trifluoromethyl)pyridin-2-yl)piperidin-4-amine (100 mg, 91%) as a white solid. LCMS (ESI, *m*/*z):* 246 [M+H]⁺.

### Step 3: Preparation of 1-(pyridin-3-yl)-3-(1-(6-(trifluoromethyl)pyridin-2-yl)piperidin-4-yl)urea

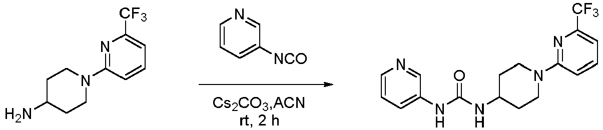

To a stirred mixture of 1-(6-(trifluoromethyl)pyridin-2-yl)piperidin-4-amine (74 mg, 0.30 mmol, 1.00 equiv) and Cs₂CO₃ (98 mg, 0.30 mmol, 1.00 equiv) in ACN (5 mL) was added 3-isocyanatopyridine (36 mg, 0.30 mmol, 1.00 equiv) in portions at rt under nitrogen atmosphere. The resulting mixture was stirred for 2 h at rt under nitrogen atmosphere. The reaction was quenched with water (20 mL) and extracted with EtOAc (3 x 20 mL). The organic layers were combined, washed with brine (20 mL), dried over anhydrous sodium sulfate, filtered and concentrated under reduced pressure. The crude product (150 mg) was purified by preparative HPLC to provide 1-(pyridin-3-yl)-3-(1-(6-(trifluoromethyl)pyridin-2-yl)piperidin-4-yl)urea (19.9 mg, 18%) as a yellow solid. ¹H NMR (400 MHz, Methanol-*d*₄) 9.32 (s, 1H), 8.33 (d, *J* = 4.8 Hz, 1H), 8.26 (d, *J* = 8.4 Hz, 1H), 7.89 (dd, Ji = 8.0 Hz, *J*₂ = 5.6 Hz, 1H), 7.69 (t, *J* = 8.0 Hz, 1H), 7.05 (d, *J* = 8.8 Hz, 1H), 6.96 (d, *J* = 7.2 Hz, 1H), 4.37 (d, *J* = 13.2 Hz, 2H), 3.95 - 3.89 (m, 1H), 3.16 - 3.08 (m, 2H), 2.04 (d, *J* = 11.6 Hz, 2H), 1.54 (dd, *J*₁ = 20.0 Hz, *J*₂ = 11.2 Hz, 2H). LCMS (ESI, *m*/*z):* 366 [M+H]⁺.

### Example 2: 1-(1-(3-Chloro-4-(4-chloro-3-(trifluoromethoxy)phenoxy)pyridin-2-yl)piperidin-4-yl)-3-(pyridin-3-yl)urea

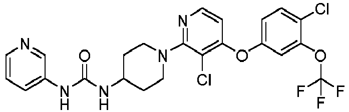

### Step 1: Preparation of t-butyl (1-(3-chloro-4-iodopyridin-2-yl)piperidin-4-yl)carbamate

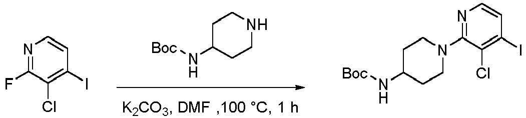

A flask was charged with 3-chloro-2-fluoro-4-iodopyridine (2.57 g, 0.01 mol, 1.00 equiv), t-butyl piperidin-4-ylcarbamate (2.00 g, 0.01 mol, 1.00 equiv), DMF (15 mL) and K₂CO₃ (2.76 g, 0.02 mmol, 2.00 equiv). The resulting solution was stirred for 1h at 100 °C, cooled to rt, and poured into 100 mL ice water. The solid was isolated by filtration and dried to provide 3.50 g (80% yield) of t-butyl (1-(3-chloro-4-iodopyridin-2-yl)piperidin-4-yl)carbamate as an off-white solid. LCMS (ESI, *m*/*z):* 438 [M+H]⁺.

### Step 2: Preparation of 1-(3-chloro-4-(4-chloro-3-(trifluoromethoxy)phenoxy)pyridin-2-yl)piperidin-4-amine

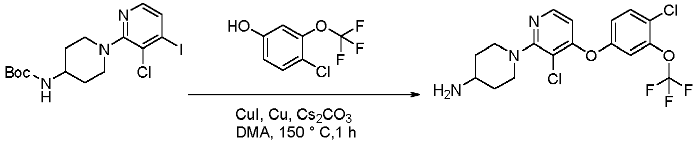

A flask was charged with t-butyl (1-(3-chloro-4-iodopyridin-2-yl)piperidin-4-yl)carbamate (400 mg, 0.91 mmol, 1.00 equiv), 4-chloro-3-(trifluoromethoxy)phenol (387 mg, 1.83 mmol, 2.00 equiv), CuI (86.8 mg, 0.46 mmol, 0.50 equiv), Cu (29.2 mg, 0.46 mmol, 0.50 equiv), Cs₂CO₃ (890 mg, 2.73 mmol, 3.00 equiv) and DMA (5 mL). The resulting solution was stirred 1 h at 150 °C before cooling to rt. The resulting solution was filtered and purified by preparative HPLC to provide 200 mg (52% yield) of 1-(3-chloro-4-(4-chloro-3-(trifluoromethoxy)phenoxy)pyridin-2-yl)piperidin-4-amine as a white solid. LCMS (ESI, *m*/*z*): 422 [M+H]⁺.

### Step 3: Preparation of 1-(1-(3-chloro-4-(4-chloro-3-(trifluoromethoxy)phenoxy)pyridin-2-yl)piperidin-4-yl)-3-(pyridin-3-yl)urea

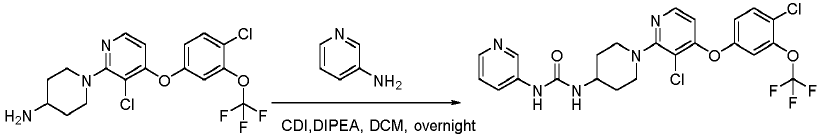

A flask was charged with pyridin-3-amine (49.0 mg, 0.52 mmol, 2.00 equiv), DIEA (67.2 mg, 0.52 mmol, 2.00 equiv), CDI (100 mg, 0.62 mmol, 2.40 equiv) and DCM (5 mL). The resulting solution was stirred for 3h at rt prior to addition of 1-(3-chloro-4-(4-chloro-3-(trifluoromethoxy)phenoxy)pyridin-2-yl)piperidin-4-amine (109 mg, 0.26 mmol, 1.00 equiv) and DIPEA (100.6 mg, 0.78 mmol, 3.00 equiv). The mixture was stirred overnight at rt. The resulting mixture was diluted with DCM (25 mL) and washed with saturated aqueous NaHCO₃ (3 x 10 mL). The organic layers was concentrated under reduced pressure. The crude product (100 mg) was purified by preparative HPLC to provide 17.3 mg (14% yield) of 1-(1-(3-chloro-4-(4-chloro-3-(trifluoromethoxy)phenoxy)pyridin-2-yl)piperidin-4-yl)-3-(pyridin-3-yl)urea as a white solid. ¹H NMR (400 MHz, Methanol-*d*₄) 8.57 (s, 1H), 8.16 (d, *J* = 6.0 Hz, 1H), 8.10 (d, *J* = 6.4 Hz, 1H), 7.98 - 7.95 (m, 1H), 7.54 (d, *J* = 10.4 Hz, 1H), 7.38 - 7.34 (m, 2H), 7.16 - 7.13 (m, 1H), 6.59 (d, *J* = 6.0 Hz, 1H), 3.85 - 3.79 (m, 3H), 3.15 - 3.04 (m, 2H), 2.10 (d, *J* = 8.8 Hz, 2 H), 1.73 - 1.70 (m, 2H). LCMS (ESI, *m*/*z*): 542 [M+H]⁺.

### Example 3: 1-(1-(5-Acetylpyridin-2-yl)piperidin-4-yl)-3-(pyridin-3-yl)urea

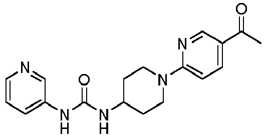

### Step 1: Preparation of t-butyl 4-(3-(pyridin-3-yl)ureido)piperidine-1-carboxylate

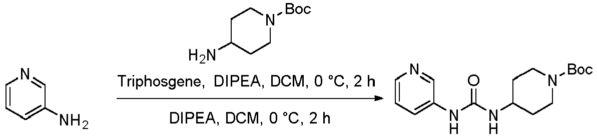

A flask was charged with pyridin-3-amine (2.35 g, 25.0 mmol, 1.00 equiv), DIPEA (6.46 g, 49.9 mmol, 2.00 equiv) and DCM (100 mL). A solution of triphosgene (2.96 g, 9.98 mmol, 0.400 equiv) was added to the reaction mixture at 0 °C. The reaction mixture was stirred at 0 °C for 2 h before concentrating under reduced pressure. The residue was dissolved into DCM (100 mL) and a solution of DIPEA (6.46 g, 49.9 mmol, 2.00 equiv) and t-butyl 4-aminopiperidine-1-carboxylate (5.00 g, 25.0 mmol, 1.00 equiv) was added to the reaction at 0 °C. The reaction mixture was stirred at 0 °C for 2 h before quenching with water (100 mL). The resulting solution was extracted with DCM (3 x 100 mL) and the organic layers were combined, washed with brine (1 × 100 mL), dried over anhydrous Na₂SO₄ and concentrated under reduced pressure. The residue was purified by reverse flash chromatography to provide 4.27 g (53% yield) of *t*-butyl 4-(3-(pyridin-3-yl)ureido)piperidine-1-carboxylate as a yellow oil. LCMS (ESI, *m*/*z*): 321 [M+H]⁺.

### Step 2: Preparation of 1-(piperidin-4-yl)-3-(pyridin-3-yl)urea

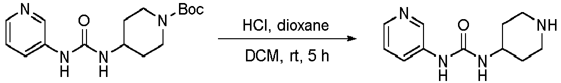

A flask was charged with t-butyl 4-(3-(pyridin-3-yl)ureido)piperidine-1-carboxylate (4.27 g, 13.3 mmol, 1.00 equiv), HCl (20 mL, 4 M in dioxane) and DCM (100 mL). The reaction mixture was stirred at rt for 5 h. The reaction was concentrated under reduced pressure to provide 3.33 g (crude) of 1-(piperidin-4-yl)-3-(pyridin-3-yl)urea as a yellow oil. LCMS (ESI, *m*/*z):* 221 [M+H]⁺.

### Step 3: Preparation of 1-(1-(5-acetylpyridin-2-yl)piperidin-4-yl)-3-(pyridin-3-yl)urea

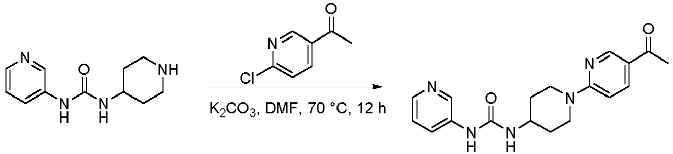

A flask was charged with 1-(piperidin-4-yl)-3-(pyridin-3-yl)urea (150 mg, 0.681 mmol, 1.00 equiv), 1-(6-chloropyridin-3-yl)ethan-1-one (96.0 mg, 0.681 mmol, 1.00 equiv), K₂CO₃ (138 mg, 1.361 mmol, 2.00 equiv) and DMF (10 mL). The reaction mixture was stirred for 12 h at 70 °C before quenching with water (20 mL). The resulting solution was extracted with EtOAc (3 x 20 mL) and the organic layers were combined, washed with brine (1 × 20 mL), dried over anhydrous Na₂SO₄ and concentrated under reduced pressure. The crude product was purified by preparative HPLC to provide 52.1 mg (17% yield) of 1-(1-(5-acetylpyridin-2-yl)piperidin-4-yl)-3-(pyridin-3-yl)urea as a yellow solid. ¹ H NMR (400 MHz, Methanol-*d*₄) δ 8.76 (d, *J* = 2.4 Hz, 1H), 8.56 (d, *J* = 2.6 Hz, 1H), 8.16 (dd, *J* = 4.8 Hz, 1.5 Hz, 1H), 8.06 (dd, *J* = 9.2 Hz, 2.4 Hz, 1H), 7.98 - 7.91 (m, 1H), 7.39 - 7.31 (m, 1H), 6.88 (d, *J* = 9.1 Hz, 1H), 4.46 (d, *J* = 13.5 Hz, 2H), 4.14 - 3.94 (m, 1H), 3.29 - 3.18 (m, 2H), 2.52 (s, 3H), 2.07 (dd, *J* = 13.1 Hz, 3.7 Hz, 2H), 1.57 - 1.43 (m, 2H). LCMS (ESI, *m*/*z):* 340 [M+H]⁺.

### Example 4: 1-(Pyridin-3-yl)-3-(1-(4-(4-(trifluoromethoxy)phenyl)pyridin-2-yl)piperidin-4-yl)urea

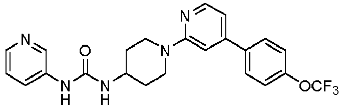

### Step 1: Preparation of 1-(1-(4-bromopyridin-2-yl)piperidin-4-yl)-3-(pyridin-3-yl)urea

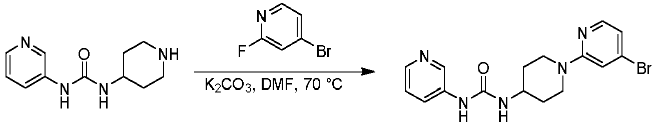

A flask was charged with 1-(piperidin-4-yl)-3-(pyridin-3-yl)urea (943 mg, 4.5 mmol, 1.5 equiv) (as prepared in Example 10, Steps 1-2), 4-bromo-2-fluoropyridine (500 mg, 3.0 mmol, 1 equiv), K₂CO₃ (789.00 mg, 6.00 mmol, 2.00 equiv) and DMF (5 mL). The reaction mixture was stirred for 2 h at 70 °C before quenching with water (20 mL). The resulting solution was extracted with EtOAc (3 x 20 mL) and the organic layers were combined, washed with brine (1 × 20 mL), dried over anhydrous Na₂SO₄ and concentrated under reduced pressure. The crude product was purified by C-18 column chromatography to provide 1-(1-(4-bromopyridin-2-yl)piperidin-4-yl)-3-(pyridin-3-yl)urea (900 mg, 84%) as a white solid. LCMS (ESI, m/z): 376 [M+H]⁺.

### Step 2: Preparation of 1-(pyridin-3-yl)-3-(1-(4-(4-(trifluoromethoxy)phenyl)pyridin-2-yl)piperidin-4-yl)urea

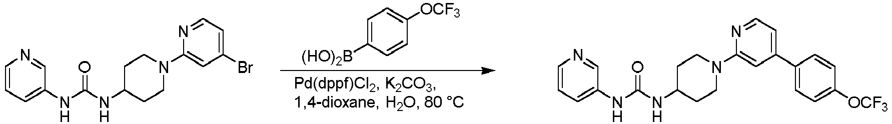

A mixture of 1-(1-(4-bromopyridin-2-yl)piperidin-4-yl)-3-(pyridin-3-yl)urea (200.00 mg, 0.53 mmol, 1.00 equiv), 4-(trifluoromethoxy)phenylboronic acid (131.35 mg, 0.63 mmol, 1.20 equiv), Pd(dppf)Cl₂ (77.79 mg, 0.10 mmol, 0.20 equiv) and K₂CO₃ (146.93 mg, 1.06 mmol, 2.00 equiv) in H₂O (1.00 mL) and dioxane (4.00 mL) was stirred for 2 h at 80 °C under nitrogen atmosphere. The resulting mixture was quenched with water (10 mL), extracted with EtOAc (3 x 10 mL) and the combined organic layers were washed with brine (2 x 10 mL), dried over anhydrous Na₂SO₄, filtered and concentrated under reduced pressure. The residue was purified by C-18 column chromatography and prep-HPLC to provide 1-(pyridin-3-yl)-3-(1-(4-(4-(trifluoromethoxy)phenyl)pyridin-2-yl)piperidin-4-yl)urea (12.20 mg, 5%) as a white solid. ¹H NMR (400 MHz, Methanol-*d*₄) *δ* 8.59 - 8.53 (m, 1H), 8.20 - 8.12 (m, 2H), 7.99 - 7.92 (m, 1H), 7.85 - 7.77 (m, 2H), 7.44 - 7.37 (m, 2H), 7.41 - 7.31 (m, 1H), 7.09 - 7.04 (m, 1H), 6.97 - 6.91 (m, 1H), 4.34 - 4.26 (m, 2H), 3.95 - 3.83 (m, 1H), 3.22 - 3.10 (m, 2H), 2.11 - 2.02 (m, 2H), 1.64 - 1.50 (m, 2H). LCMS (ESI, *m*/*z*): 458 [M+H]⁺.

### Example 5: 1-(1-(3-Chloro-4-(4-fluoro-3-(trifluoromethoxy)phenoxy)pyridin-2-yl)piperidin-4-yl)-3-(pyridin-3-yl)urea

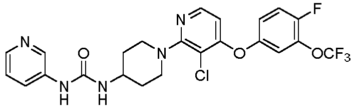

### Step 1: Preparation of 1-(1-(4-bromo-3-chloropyridin-2-yl)piperidin-4-yl)-3-(pyridin-3-yl)urea

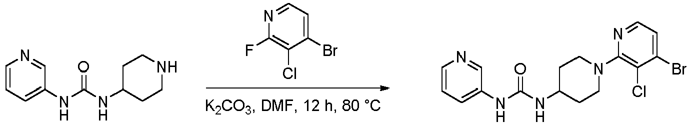

A flask was charged with 4-bromo-3-chloro-2-fluoropyridine (1.00 g, 4.76 mmol, 1.00 equiv), 1-(piperidin-4-yl)-3-(pyridin-3-yl)urea (1.15 g, 5.24 mmol, 1.10 equiv) (as prepared in Example 3, Steps 1-2), K₂CO₃ (1.98 g, 14.3 mmol, 3.00 equiv) and DMF (15 mL). The reaction mixture was stirred for 12 h at 80 °C before quenching with water (30 mL). The resulting solution was extracted with EtOAc (3 x 30 mL) and the organic layers were combined, washed with brine (1 × 30 mL), dried over anhydrous Na₂SO₄ and concentrated under reduced pressure. The residue was purified by reverse flash chromatography to provide 750 mg (38% yield) of 1-(1-(4-bromo-3-chloropyridin-2-yl)piperidin-4-yl)-3-(pyridin-3-yl)urea as a white solid. LCMS (ESI, *m*/*z):* 410 [M+H]⁺.

### Step 2: Preparation of 1-(1-(3-chloro-4-(4-fluoro-3-(trifluoromethoxy)phenoxy)pyridin-2-yl)piperidin-4-yl)-3-(pyridin-3-yl)urea

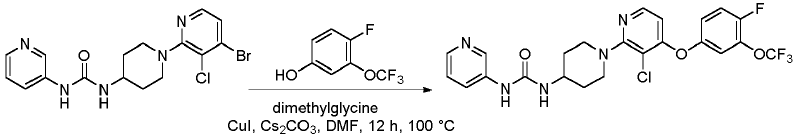

A flask was charged with 1-(1-(4-bromo-3-chloropyridin-2-yl)piperidin-4-yl)-3-(pyridin-3-yl)urea (65.0 mg, 0.158 mmol, 1.00 equiv), 4-fluoro-3-(trifluoromethoxy)phenol (34.0 mg, 0.174 mmol, 1.10 equiv), Cs₂CO₃ (155 mg, 0.474 mmol, 3.00 equiv), dimethylglycine (5.00 mg, 0.0474 mmol, 0.300 equiv), CuI (3.00 mg, 0.0158 mmol, 0.100 equiv) and DMF (3 mL). The reaction mixture was stirred for 12 h at 100 °C under N₂, diluted with EtOAc (50 mL), and filtered. The filtrate was washed with brine (3 x 20 mL), dried over anhydrous Na₂SO₄ and concentrated under reduced pressure. The residue was purified by preparative HPLC to provide 7.4 mg (9% yield) of 1-(1-(3-chloro-4-(4-fluoro-3-(trifluoromethoxy)phenoxy)pyridin-2-yl)piperidin-4-yl)-3-(pyridin-3-yl)urea as an off-white solid. ¹H NMR (400 MHz, DMSO-*d₆*) δ 8.54 (d, *J* = 2.5 Hz, 2H), 8.11 (t, *J* = 5.3 Hz, 2H),7.93 - 7.85 (m, 1H), 7.68 - 7.55 (m, 2H), 7.33 - 7.28 (m, 1H), 7.26 (dd, *J* = 8.3, 4.7 Hz, 1H), 6.51 (d, *J* = 5.6 Hz, 1H), 6.43 (d, *J* = 7.7 Hz, 1H), 3.75 - 3.64 (m, 3H), 3.00 (t, *J* = 11.5 Hz, 2H), 1.96 (d, *J* = 12.6 Hz, 2H), 1.66 - 1.51 (m, 2H). LCMS (ESI, *m*/*z*): 526[M+H]⁺.

### Example 6: 1-(1-(3-Chloro-4-(2-chloro-4-ethynylphenoxy)pyridin-2-yl)piperidin-4-yl)-3-(pyridin-3-yl)urea

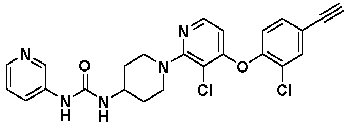

### Step 1: Preparation of 4-(4-bromo-2-chlorophenoxy)-2,3-dichloropyridine

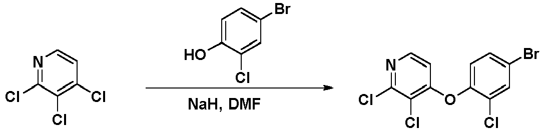

A mixture of 4-bromo-2-chlorophenol (632 mg, 3.0 mmol, 1.0 equiv) and NaH (123 mg, 3.0 mmol, 1.0 equiv) in DMF (5 mL) was stirred at rt for 30 min, and 2,3,4-trichloropyridine (500 mg, 2.70 mmol, 0.90 equiv) was then added. The reaction mixture was stirred for 2 h at 100 °C, quenched with water (10 mL), extracted with EtOAc (3 x 10 mL) and the combined organic layers were washed with brine (2 x 10 mL), dried over anhydrous Na₂SO₄, filtered and concentrated under reduced pressure. The residue was purified by C-18 column chromatography to provide 4-(4-bromo-2-chlorophenoxy)-2,3-dichloropyridine (500 mg, 46%) as a yellow solid. LCMS (ESI, *m*/*z*): 352 [M+H]⁺.

### Step 2: Preparation of t-butyl (1-(4-(4-bromo-2-chlorophenoxy)-3-chloropyridin-2-yl)piperidin-4-yl)carbamate

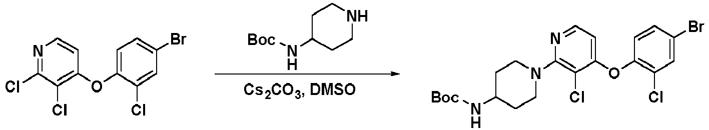

A mixture of 4-(4-bromo-2-chlorophenoxy)-2,3-dichloropyridine (300 mg, 0.84 mmol, 1.0 equiv) t-butyl N-(piperidin-4-yl)carbamate (510 mg, 2.54 mmol, 3.0 equiv) and Cs₂CO₃ (443 mg, 1.35 mmol, 1.60 equiv) in DMSO (5.00 mL) was stirred overnight at 100 °C and quenched with water (10 mL). The resulting mixture was extracted with EtOAc (3 x 10 mL) and the combined organic layers were washed with brine (2 x 10 mL), dried over anhydrous Na₂SO₄, filtered and concentrated under reduced pressure. The residue was purified by C-18 column chromatography to provide t-butyl (1-(4-(4-bromo-2-chlorophenoxy)-3-chloropyridin-2-yl)piperidin-4-yl)carbamate (120.00 mg, 27%) as a yellow oil. LCMS (ESI, *m*/*z):* 516 [M+H]⁺.

### Step 3: Preparation of t-butyl (1-(3-chloro-4-(2-chloro-4-((trimethylsilyl)ethynyl)phenoxy)pyridin-2-yl)piperidin-4-yl)carbamate

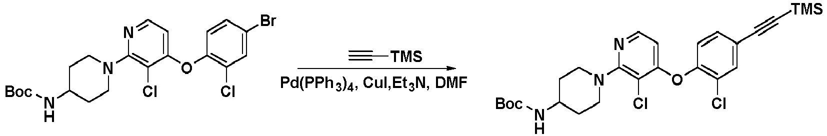

A mixture of t-butyl (1-(4-(4-bromo-2-chlorophenoxy)-3-chloropyridin-2-yl)piperidin-4-yl)carbamate (100 mg, 0.19 mmol, 1.00 equiv), trimethylsilylacetylene (190 mg, 1.93 mmol, 10.00 equiv), Pd(PPh₃)₄ (45 mg, 0.039 mmol, 0.2 equiv), CuI (7 mg, 0.039 mmol, 0.2 equiv) and Et₃N (59 mg, 0.58 mmol, 3 equiv) in DMF (2.00 mL) was stirred overnight at 100 °C under N₂. The mixture was diluted with water (20 mL) and extracted with EtOAc (3 x 10 mL) and the combined organic layers were washed with brine (2 x 10 mL), dried over anhydrous Na₂SO₄, filtered and concentrated under reduced pressure. The residue was purified by column chromatography to provide *t*-butyl (1-(3-chloro-4-(2-chloro-4-((trimethylsilyl)ethynyl)phenoxy)pyridin-2-yl)piperidin-4-yl)carbamate (100 mg, 97%) as a yellow solid. LCMS (ESI, *m*/*z*)*:* 534 [M+H]⁺.

### Step 4: Preparation of 1-(3-chloro-4-(2-chloro-4-ethynylphenoxy)pyridin-2-yl)piperidin-4-amine

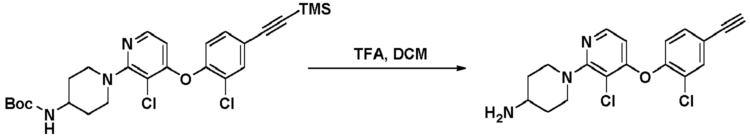

A mixture of *t*-butyl (1-(3-chloro-4-(2-chloro-4-((trimethylsilyl)ethynyl)phenoxy)pyridin-2-yl)piperidin-4-yl)carbamate (100 mg, 0.18 mmol, 1.00 equiv) and TFA (1 mL) in DCM (2 mL) was stirred for 2 h at rt. The mixture was concentrated under reduced pressure to provide 1-(3-chloro-4-(2-chloro-4-ethynylphenoxy)pyridin-2-yl)piperidin-4-amine (60 mg, crude) as a yellow oil. LCMS (ESI, *m*/*z):* 362 [M+H]⁺.

### Step 5: Preparation of 1-(1-(3-chloro-4-(2-chloro-4-ethynylphenoxy)pyridin-2-yl)piperidin-4-yl)-3-(pyridin-3-yl)urea

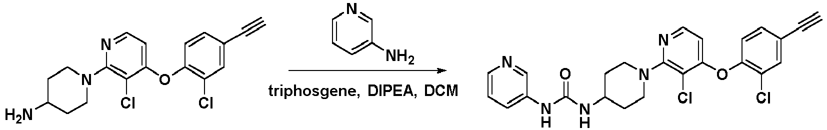

To a mixture of 3-aminopyridine (16 mg, 0.20 mmol, 1.0 equiv) and triphosgene (29 mg, 0.10 mmol, 0.60 equiv) in DCM (5 mL), was added DIPEA (64 mg, 0.60 mmol, 3.0 equiv) dropwise at 0 °C under an atmosphere of nitrogen. The reaction mixture was stirred for 1 h at 0 °C prior to addition of a mixture of 1-(3-chloro-4-(2-chloro-4-ethynylphenoxy)pyridin-2-yl)piperidin-4-amine (60.00 mg, 0.20 mmol, 1.00 equiv) in DCM (3 mL). The reaction mixture was stirred for another 1 h at 0 °C and quenched with water (10 mL). The resulting mixture was extracted with DCM (3 x 10 mL) and the combined organic layers were washed with brine (2 x 10 mL), dried over anhydrous Na₂SO₄, filtered and concentrated under reduced pressure. The residue was purified by C-18 column chromatography and preparative HPLC to give 1-(1-(3-chloro-4-(2-chloro-4-ethynylphenoxy)pyridin-2-yl)piperidin-4-yl)-3-(pyridin-3-yl)urea (8.20 mg, 10%) as a white solid. ¹H NMR (400 MHz, Methanol-*d*₄) *δ* 8.59 - 8.54 (m, 1H), 8.19 - 8.13 (m, 1H), 8.06 - 8.00 (m, 1H), 8.00 - 7.93 (m, 1H), 7.72 - 7.67 (m, 1H), 7.55 - 7.48 (m, 1H), 7.40 - 7.32 (m, 1H), 7.22 - 7.15 (m, 1H), 6.37 - 6.31 (m, 1H), 3.87 - 3.77 (m, 3H), 3.69 - 3.65 (m, 1H), 3.13 - 3.02 (m, 2H), 2.14 - 2.03 (m, 2H), 1.80 - 1.60 (m, 2H).LCMS (ESI, *m*/*z*): 482 [M+H]⁺.

### Example 7: 1-(Pyridin-3-yl)-3-(1-(4-(4-(trifluoromethyl)phenoxy)pyridin-2-yl)piperidin-4-yl)urea

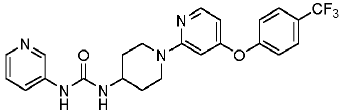

### Step 1: Preparation of 2,3-dichloro-4-(4-(trifluoromethyl)phenoxy)pyridine

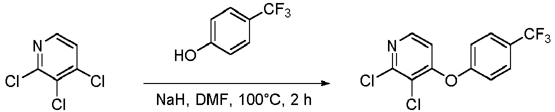

A flask was charged with 4-(trifluoromethyl)phenol (402 mg, 2.48 mmol, 1.00 equiv), NaH (60 mg, 2.48 mmol, 1.00 equiv) and DMF (10 mL). The reaction mixture was stirred for 0.5 h at rt. A solution of 2,3,4-trichloropyridine (406 mg, 2.23 mmol, 0.900 equiv) was added to the reaction mixture. The reaction mixture was stirred at 100 °C for 2 h before quenching with water (30 mL). The resulting solution was extracted with DCM (3 x 30 mL) and the organic layers were combined, washed with brine (1 × 30 mL), dried over anhydrous Na₂SO₄ and concentrated under reduced pressure. The residue was purified by reverse flash chromatography to provide 500 mg (65% yield) of 2,3-dichloro-4-(4-(trifluoromethyl)phenoxy)pyridine as a white solid. LCMS (*ESI, m*/*z*)i*:* 308 [M+H]⁺.

### Step 2: Preparation of t-butyl (1-(3-chloro-4-(4-(trifluoromethyl)phenoxy)pyridin-2-yl)piperidin-4-yl)carbamate

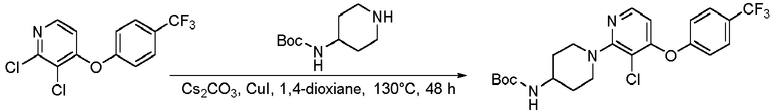

A flask was charged with 2,3-dichloro-4-(4-(trifluoromethyl)phenoxy)pyridine (480 mg, 1.56 mmol, 1.00 equiv), *t*-butyl piperidin-4-ylcarbamate (342 mg, 1.71 mmol, 1.10 equiv), Cs₂CO₃ (1.53 g, 4.68 mmol, 3.00 equiv), CuI (29 mg, 0.156 mmol, 0.100 equiv) and 1,4-dioxane (10 mL). The reaction mixture was stirred at 130 °C for 48 h before quenching with water (30 mL). The resulting solution was extracted with DCM (3 x 30 mL) and the organic layers were combined, washed with brine (1 x 30 mL), dried over anhydrous Na₂SO₄ and concentrated under reduced pressure. The residue was purified by reverse flash chromatography to provide 400 mg (54% yield) of *t*-butyl (1-(3-chloro-4-(4-(trifluoromethyl)phenoxy)pyridin-2-yl)piperidin-4-yl)carbamate as a white solid. LCMS (ESI, *m*/*z):* 472 [M+H]⁺.

### Step 3: Preparation of t-butyl (1-(4-(4-(trifluoromethyl)phenoxy)pyridin-2-yl)piperidin-4-yl)carbamate

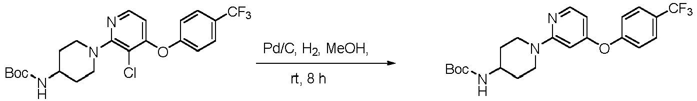

A flask was charged with *t*-butyl (1-(3-chloro-4-(4-(trifluoromethyl)phenoxy)pyridin-2-yl)piperidin-4-yl)carbamate (380 mg, 0.805 mmol, 1.00 equiv), Pd/C (19.0 mg, 0.161 mmol, 0.200 equiv) and MeOH (10 mL). The reaction mixture was stirred at rt for 8 h under H₂ atmosphere. The resulting solution was filtered, and the filter cake was washed with EtOAc (3 x 10 mL), the filtrate was concentrated under reduced pressure to provide t-butyl (1-(4-(4-(trifluoromethyl)phenoxy)pyridin-2-yl)piperidin-4-yl)carbamate (300 mg crude) as a yellow oil. LCMS (ESI, *m*/*z):* 438 [M+1]⁺.

### Step 4: Preparation of -(4-(4-(trifluoromethyl)phenoxy)pyridin-2-yl)piperidin-4-amine

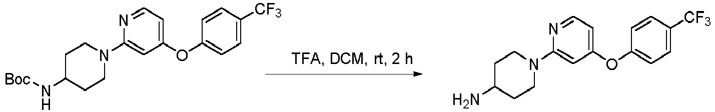

A flask was charged with t-butyl (1-(4-(4-(trifluoromethyl)phenoxy)pyridin-2-yl)piperidin-4-yl)carbamate (300 mg, 0.686 mmol, 1.00 equiv), TFA (3mL) and DCM (10 mL). The reaction mixture was stirred overnight at rt. The pH value of the solution was adjusted to 8-9 with NaHCO₃. The resulting mixture was extracted with DCM (3 x 30 mL) and the organic layers were combined, washed with brine (1 x 30 mL), dried over anhydrous Na₂SO₄ and concentrated under reduced pressure. The residue was purified by reverse flash chromatography provide 100 mg (43% yield) of 1-(4-(4-(trifluoromethyl)phenoxy)pyridin-2-yl)piperidin-4-amine as a yellow oil. LCMS (ESI, *m*/*z):* 338 [M+H]⁺.

### Step 5: Preparation of 1-(pyridin-3-yl)-3-(1-(4-(4-(trifluoromethyl)phenoxy)pyridin-2-yl)piperidin-4-yl)urea

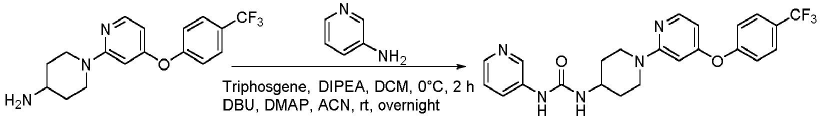

A flask was charged with pyridin-3-amine (31 mg, 0.326 mmol, 1.10 equiv) and DIPEA (77.0 mg, 0.594 mmol, 2.00 equiv) in DCM (2 mL). A solution of triphosgene (44.0 mg, 0.148 mmol, 0.500 equiv) in DCM (4 mL) was added dropwise at 0 °C. The mixture was then stirred for 2 hours at rt and concentrated under vacuum. To a solution of 1-(4-(4-(trifluoromethyl)phenoxy)pyridin-2-yl)piperidin-4-amine (100 mg, 0.297 mmol, 1.00 equiv) in ACN (3 mL) was added the above residue, followed by DBU (181 mg, 1.19 mmol, 4.00 equiv) and DMAP (7.00 mg, 0.0594 mmol, 0.200 equiv). The mixture was stirred overnight at rt and concentrated under vacuum. The resulting mixture was extracted with DCM (3 x 20 mL) and the combined organic layers were washed with brine (1 × 20 mL), dried over anhydrous Na₂SO₄, concentrated under reduced pressure. The crude product was purified by preparative HPLC to provide 44 mg (33% yield) of 1-(pyridin-3-yl)-3-(1-(4-(4-(trifluoromethyl)phenoxy)pyridin-2-yl)piperidin-4-yl)urea as a white solid . ¹H NMR (400 MHz, DMSO-*d₆*) δ 8.57 - 8.46 (m, 2H), 8.15 - 8.03 (m, 2H), 7.92 - 7.84 (m, 1H), 7.80 (d, *J* = 8.4 Hz, 2H), 7.35 - 7.20 (m, 3H), 6.54 (d, *J* = 2.0 Hz, 1H), 6.36 (d, *J* = 7.6 Hz, 1H), 6.27 (dd, *J* = 5.7, 1.9 Hz, 1H), 4.12 (dd, *J* = 11.5, 7.0 Hz, 2H), 3.84 - 3.65 (m, 1H), 3.12 - 2.95 (m, 2H), 1.87 (dd, *J* = 13.0, 3.8 Hz, 2H), 1.45 - 1.30 (m, 2H). LCMS (ESI, *m*/*z):* 458 [M+H]⁺.

### Examples 8-29: Examples 8-29 were prepared in a similar manner to Examples 1-7.

| **Ex** | **Name** | **Structure** | **¹H NMR (CDCl₃, 300 MHz or 400 MHz)** | **LRMS [M+H]⁺** |
|---|---|---|---|---|
| **8** | 1-(1-(3-Chloro-4-(trifluoromethyl)pyr idin-2-yl)piperidin-4-yl)-3-(pyridin-3-yl)urea | 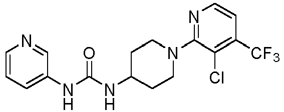 | δ 9.17 (s, 1H), 8.37 (d, *J* = 4.8 Hz, 2H), 8.25 (d, *J* = 8.8 Hz, 1H), 7.88 - 7.85 (m, 1H), 7.31 (d, *J* = 5.2 Hz, 1H), 3.91 - 3.79 (m, 3H), 3.11 - 3.05 (m, 2H), 2.12 - 2.06 (m, 2H), 1.80 - 1.70 (m, 2H). LCMS (ESI, *m*/*z*): 400 [M+H]⁺. | 400 |
| **9** | 1-(1-(3-Methyl-4-(4-(trifluoromethoxy)p henoxy)pyridin-2-yl)piperidin-4-yl)-3-(pyridin-3-yl)urea | 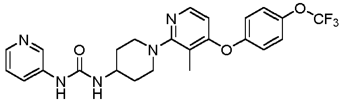 | δ 8.55 (s, 1H), 8.28 (d, *J* = 8.8 Hz, 2H), 8.06 (d, *J* = 5.6 Hz, 1H), 7.85 (s, 1H), 7.35 (s, 1H), 7.26 (s, 1H), 7.09 - 7.06 (m, 2H), 6.39 (d, *J* = 6.0 Hz, 1H), 5.52 - 5.50 (m, 1H), 3.99 (s, 1H), 3.54 - 3.51 (m, 2H), 3.09 (t, *J* = 10.4 Hz, 2H), 2.25 (s, 3H), 2.20 - 2.13 (m, 2H), 1.79 - 1.63 (m, 2H) | 488 |
| **10** | 1-(1-(3-Chloro-4-(4-(trifluoromethoxy)p henoxy)pyridin-2-yl)piperidin-4-yl)-3-(pyridin-3-yl)urea | 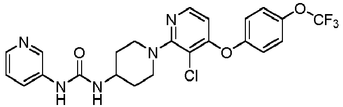 | 8.57 (s, 1H), 8.15 (d, *J* = 4.4 Hz, 1H), 8.10 (d, *J* = 5.6 Hz, 1H), 7.98 - **7.95** (m 1H), 7.41 - 7.35 (m, 3H), 7.23 - 7.20 (m, 2H), 6.48 (d, *J* = 5.6 Hz, 1H), 3.87 - 3.78 (m, 3H), 3.09 - 3.03 (m, 2H), 2.12 - 2.08 (m, 2H), 1.78 - 1.67 (m, 2H) | 508 |
| **11** | 1-(1-(5-Chloropyridin-2-yl)piperidin-4-yl)-3-(pyridin-3-yl)urea | 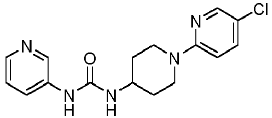 | δ 8.56 (dd, *J* = 2.6, 0.8 Hz, 1H), 8.15 (dd, *J* = 4.8, 1.4 Hz, 1H), 8.05 (dd, *J* = 2.7, 0.7 Hz, 1H), 7.95 (m,1H), 7.52 (dd, *J* = 9.1, 2.7 Hz, 1H), 7.35 (m, 1H), 6.87 - 6.82 (m, 1H), 4.25 - 4.13 (m, 2H), 3.86 (m, 1H), 3.09 (m, 2H), 2.02 (m, 2H), 1.57 - 1.44 (m, 2H) | 332 |
| **12** | 1-(1-(4-Cyanopyridin-2-yl)piperidin-4-yl)-3-(pyridin-3-yl)urea | 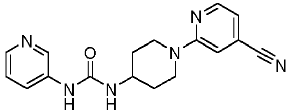 | δ 8.56 (d, *J* = 2.6 Hz, 1H), 8.26 (d, *J* = 5.1 Hz, 1H), 8.15 (d, *J* = 4.8 Hz, 1H), 8.00 - 7.91 (m, 1H), 7.35 (dd, *J* = 8.4, 4.8 Hz, 1H), 7.16 (s, 1H), 6.82 (d, *J* = 5.0 Hz, 1H), 4.31 (d, *J* = 13.6 Hz, 2H), 3.90 (t, *J* = 10.3 Hz, 1H), 3.15 (t, *J* = 12.0 Hz, 2H), 2.09 - 1.99 (m, 2H), 1.57 - 1.44 (m, 2H) | 323 |
| **13** | 1-(1-(3-Chloro-4-(2-(trifluoromethoxy)p henoxy)pyridin-2-yl)piperidin-4-yl)-3-(pyridin-3-yl)urea | 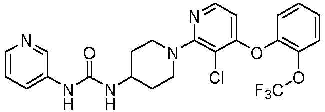 | (DMSO-*d₆*) δ 8.62 - 8.50 (m, 2H), 8.10 (dd, *J* = 14.1, 5.1 Hz, 2H), 7.89 (d, *J* = 8.3 Hz, 1H), 7.61 (d, *J* = 8.1 Hz, 1H), 7.55 - 7.47 (m, 1H), 7.47 - 7.35 (m, 2H), 7.26 (dd, *J* = 8.4, 4.7 Hz, 1H), 6.42 (dd, *J* = 23.7, 6.6 Hz, 2H), 3.82 - 3.61 (m, 3H), 3.07 - 2.94 (m, 2H), 2.02 - 1.90 (m, 2H), 1.66 - 1.51 (m, 2H) | 508 |
| **14** | 1-(1-(3-Chloro-4-(3-fluoro-4-(trifluoromethoxy)p henoxy)pyridin-2-yl)piperidin-4-yl)-3-(pyridin-3-yl)urea | 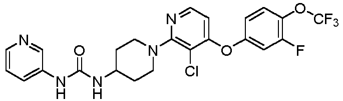 | (DMSO-*d₆*) δ 8.54 (d, *J* = 2.4 Hz, 2H), 8.17 - 8.08 (m, 2H), 7.92 - 7.84 (m, 1H), 7.67 (t, *J* = 8.9 Hz, 1H), 7.50 (dd, *J* = 11.1, 2.9 Hz, 1H), 7.26 (dd, *J* = 8.4, 4.7 Hz, 1H), 7.14 -7.06 (m, 1H), 6.66 (d, *J* = 5.5 Hz, 1H), 6.43 (d, *J* = 7.7 Hz, 1H), 3.69 (d, *J* = 13.8 Hz, 3H), 3.01 (t, *J* = 11.7 Hz, 2H), 1.96 (d, *J* = 12.5 Hz, 2H), 1.64 - 1.51 (m, 2H) | 526 |
| **15** | 1-(1-(3-Chloro-4-(3-chloro-4-(trifluoromethoxy)p henoxy)pyridin-2-yl)piperidin-4-yl)-3 - (pyridin-3-yl)urea | 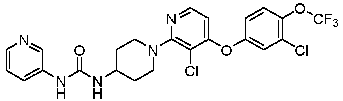 | (DMSO-*d₆*) δ 8.54 (d, *J* = 2.7 Hz, 2H), 8.13 (dd, *J* = 7.9, 5.4 Hz, 2H), 7.89 (dd, *J* = 8.5, 2.2 Hz, 1H), 7.69 - 7.60 (m, 2H), 7.30 - 7.22 (m, 2H), 6.65 (d, *J* = 5.6 Hz, 1H), 6.43 (d, *J* = 7.8 Hz, 1H), 3.69 (d, *J* = 13.9 Hz, 2H), 3.01 (t, *J* = 11.8 Hz, 2H), 1.96 (d, *J* = 12.4 Hz, 2H),1.65 - 1.53 (m, 2H) | 542 |
| **16** | 1-(1-(3-Chloro-4-(2-methyl-4-(trifluoromethoxy)p henoxy)pyridin-2-yl)piperidin-4-yl)-3 - (pyridin-3-yl)urea | 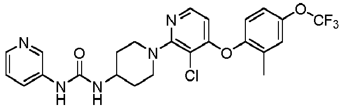 | (DMSO-*d₆*) δ 8.56 - 8.50 (m, 2H), 8.12 (dd, *J* = 4.6, 1.5 Hz, 1H), 8.06 (d, *J* = 5.6 Hz, 1H), 7.92 - 7.87 (m, 1H), 7.45 (d, *J* = 2.4 Hz, 1H), 7.33 - 7.18 (m, 3H), 6.42 (d, *J* = 7.8 Hz, 1H), 6.29 (d, *J* = 5.6 Hz, 1H), 3.69 (d, *J* = 13.2 Hz, 3H), 3.01 (t, *J* = 11.2 Hz, 2H), 2.18 (s, 3H), 1.97 (d, *J* = 12.6 Hz, 2H), 1.65 - 1.52 (m, 2H) | 522 |
| **17** | 1-(1-(3-Chloro-4-(3-(trifluoromethoxy)p henoxy)pyridin-2-yl)piperidin-4-yl)-3-(pyridin-3-yl)urea | 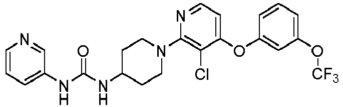 | (DMSO-*d₆*) δ 8.54 (d, *J* = 3.4 Hz, 2H), 8.16 - 8.09 (m, 2H), 7.93 - 7.85 (m, 1H), 7.60 (t, *J* = 8.3 Hz, 1H), 7.32 - 7.22 (m, 3H), 7.19 (dd, *J* = 8.2, 2.4 Hz, 1H), 6.55 (d, *J* = 5.6 Hz, 1H), 6.42 (d, *J* = 7.7 Hz, 1H), 3.69 (d, *J* = 13.4 Hz, 3H), 3.01 (t, *J* = 11.8 Hz, 2H), 1.97 (d, *J* = 12.7 Hz, 2H), 1.66 - 1.53 (m, 2H) | 508 |
| **18** | 1-(1-(4-Methoxypyridin-2-yl)piperidin-4-yl)-3-(pyridin-3-yl)urea | 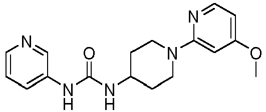 | δ 8.40 - 8.34 (m, 1H), 8.26 - 8.20 (m, 1H), 8.08 - 7.99 (m, 2H), 7.66 - 7.61 (m, 1H), 7.27 - 7.19 (m, 1H), 6.32 - 6.25 (m, 1H), 6.16 - 6.11 (m, 1H), 5.47 - 5.41 (m, 1H), 4.16 - 4.08 (m, 2H), 4.00 - 3.88 (m, 1H), 3.86 - 3.81 (m, 3H), 3.09 - 2.97 (m, 2H), 2.12 - 2.02 (m, 2H), 1.51 - 1.35 (m, 2H) | 328 |
| **19** | 1-(1-(4-(Cyclopropylmetho xy)pyridin-2-yl)piperidin-4-yl)-3-(pyridin-3-yl)urea | 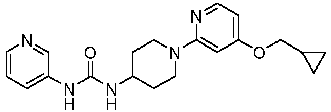 | (DMSO-*d₆*) δ 8.61 (s, 1H), 8.54 (d, *J* = 2.6 Hz, 1H), 8.12 (dd, *J* = 4.6, 1.5 Hz, 1H), 7.97 - 7.81 (m, 2H), 7.25 (dd, *J* = 8.3, 4.7 Hz, 1H), 6.42 (d, *J* = 7.6 Hz, 1H), 6.33 - 6.17 (m, 2H), 4.27 - 4.04 (m, 2H), 3.87 (d, *J* = 7.1 Hz, 2H), 3.80 - 3.64 (m, 1H), 3.13 - 2.95 (m, 2H), 1.86 (dd, *J* = 13.2, 3.8 Hz, 2H), 1.45 - 1.28 (m, 2H), 1.27 - 1.12 (m, 1H), 0.61 - 0.53 (m, 2H), 0.42 - 0.24 (m, 2H) | 368 |
| **20** | 1-(1-(3-Cyano-4-(4-(trifluoromethyl)phe noxy)pyridin-2-yl)piperidin-4-yl)-3-(pyridin-3-yl)urea | 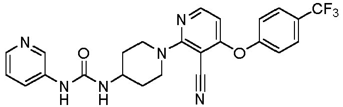 | (DMSO-*d₆*) δ 8.65 - 8.46 (m, 2H), 8.25 (d, *J* = 5.8 Hz, 1H), 8.13 (dd, *J* = 4.6, 1.5 Hz, 1H), 7.96 - 7.80 (m, 3H), 7.50 (d, *J* = 8.4 Hz, 2H), 7.26 (dd, *J* = 8.3, 4.6 Hz, 1H), 6.44 (d, *J* = 7.7 Hz, 1H), 6.29 (d, *J* = 5.7 Hz, 1H), 4.22 (d, *J* = 13.5 Hz, 2H), 3.82 (d, *J* = 7.1 Hz, 1H), 3.30 - 3.21 (m, 2H), 1.98 (d, *J* = 12.4 Hz, 2H), 1.54 (q, *J =* 9.7 Hz, 2H) | 483 |
| **21** | 1-(1-(3-Chloro-4-((tetrahydro-2H-pyran-4-yl)oxy)pyridin-2-yl)piperidin-4-yl)-3 - (pyridin-3-yl)urea | 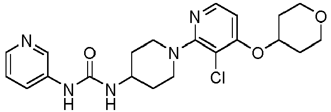 | (DMSO-*d₆*) δ 8.59 (s, 1H), 8.54 (d, *J* = 2.6 Hz, 1H), 8.11 (dd, *J* = 4.6, 1.5 Hz, 1H), 8.07 (d, *J* = 5.6 Hz, 1H), 7.91 - 7.85 (m, 1H), 7.25 (dd, *J* = 8.3, 4.6 Hz, 1H), 6.94 (d, *J* = 5.7 Hz, 1H), 6.45 (d, *J* = 7.7 Hz, 1H), 4.87 - 4.75 (m, 1H), 3.89 - 3.79 (m, 2H), 3.76 - 3.65 (m, 1H), 3.63 - 3.46 (m, 4H), 2.99 - 2.86 (m, 2H), 2.04 -1.88 (m, 4H), 1.72 - 1.60 (m, 2H), 1.60 - 1.47 (m, 2H) | 432 |
| **22** | Isopropyl 4-((2-(4-(3-(pyridin-3-yl)ureido)piperidin-1-yl)pyridin-4-yl)oxy)piperidine-1-carboxylate | 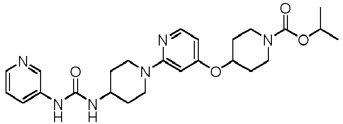 | (Metlianol-*d*₄) δ 8.56 (dd, *J* = 2.7 Hz, 0.7 Hz, 1H), 8.15 (dd, *J* = 4.9 Hz, 1.4 Hz, 1H), 7.98 - 7.90 (m, 2H), 7.38 - 7.3 7.31 (m, 1H), 6.38 (dd, *J* = 5.9 Hz, 2.1 Hz, 1H), 6.35 (d, *J* = 2.1 Hz, 1H), 4.94 - 4.88 (m, 1H), 4.78 - 4.65 (m, 1H), 4.19 - 4.08 (m, 2H), 3.90 - 3.80 (m, 1H), 3.79 - 3.68 (m, 2H), 3.49 - 3.38 (m, 2H), 3.12 - 3.01 (m, 2H), 2.08 - 1.91 (m, 4H), 1.77 - 1.64 (m, 2H), 1.60 - 1.45 (m, 2H), 1.27 (d, *J=* 6.2 Hz, 6H) | 483 |
| **23** | 1-(1-(3-(2-Chloro-4-(trifluoromethoxy)p henoxy)pyridin-2-yl)piperidin-4-yl)-3 - (pyridin-3-yl)urea | 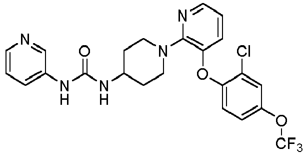 | (Methanol-*d*₄) δ 8.53 (dd, *J* = 2.6 Hz, 0.8 Hz, 1H), 8.17 - 8.08 (m, 2H), 7.97 - 7.90 (m, 1H), 7.54 (dd, *J* = 2.9 Hz, 0.9 Hz, 1H), 7.37 - 7.31 (m, 2H), 7.24 - 7.17 (m, 1H), 6.99 (dd, *J* = 7.8 Hz, 4.9 Hz, 1H), 6.75 (d, *J* = 9.1 Hz, 1H), 3.98 - 3.86 (m, 2H), 3.81 - 3.65 (m, 1H), 3.11 - 2.90 (m, 2H), 2.00 - 1.87 (m, 2H), 1.45 - 1.21 (m, 2H) | 508 |
| **24** | 1-(1-(3-Chloro-6-(2-chloro-4-(trifluoromethoxy)p henoxy)pyridin-2-yl)piperidin-4-yl)-3-(pyridin-3-yl)urea | 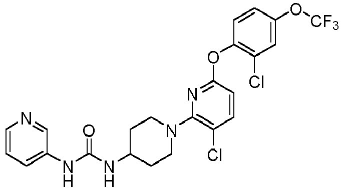 | (Methanol-*d*₄) δ 8.54 (d, *J* = 2.6 Hz, 1H), 8.15 (dd, *J* = 4.8 Hz, 1.4 Hz, 1H), 7.98 - 7.89 (m, 1H), 7.71 (d, *J* = 8.3 Hz, 1H), 7.56 - 7.47 (m, 1H), 7.41 - 7.29 (m, 3H), 6.60 (d, *J* = 8.3 Hz, 1H), 3.76 - 3.60 (m, 3H), 2.91 - 2.76 (m, 2H), 1.92 - 1.80 (m, 2H), 1.54 - 1.38 (m, 2H) | 542 |
| **25** | 1-(1-(5-Ethynylpyridin-2-yl)piperidin-4-yl)-3-(pyridin-3-yl)urea | 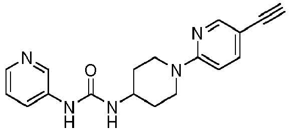 | (DMSO-*d₆*) δ 8.64 - 8.45 (m, 2H), 8.22 (d, *J* = 2.3 Hz, 1H), 8.11 (dd, *J* = 4.7, 1.5 Hz, 1H), 7.93 - 7.77 (m, 1H), 7.57 (dd, *J* = 8.9, 2.4 Hz, 1H), 7.25 (dd, *J* = 8.3, 4.7 Hz, 1H), 6.85 (d, J = 8.9 Hz, 1H), 6.35 (d, *J* = 7.7 Hz, 1H), 4.35 - 4.14 (m, 2H), 4.06 (s, 1H), 3.87 - 3.63 (m, 1H), 3.16 - 2.95 (m, 2H), 1.88 (dd, *J* = 13.0, 3.8 Hz, 2H), 1.49 - 1.24 (m, 2H) | 322 |
| **26** | 1-(1-(3-Chloro-4-(3-(trifluoromethoxy)p henyl)pyridin-2-yl)piperidin-4-yl)-3-(pyridin-3-yl)urea | 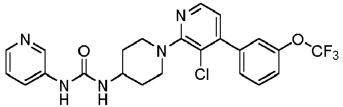 | (Methanol-*d*₄) δ 8.57 (d, *J* = 2.6 Hz, 1H), 8.23 (d, *J* = 5.0 Hz, 1H), 8.15 (dd, *J* = 4.8, 1.4 Hz, 1H), 7.99 - 7.94 (m, 1H), 7.63 - 7.57 (m, 1H), 7.48 - 7.43 (m, 1H), 7.41 - 7.37 (m, 2H), 7.37 - 7.33 (m, 1H), 7.00 (d, *J* = 5.0 Hz, 1H), 3.90 - 3.73 (m, 3H), 3.13 - 3.02 (m, 2H), 2.10 (dd, *J* = 12.2, 3.8 Hz, 2H), 1.78 - 1.66 (m, 2H) | 492 |
| **27** | 1-(1-(3-Chloro-4-(4-(trifluoromethoxy)p henyl)pyridin-2-yl)piperidin-4-yl)-3-(pyridin-3-yl)urea | 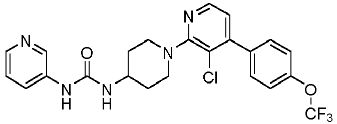 | (Methanol-*d*₄) δ 8.57 (d, *J* = 2.6 Hz, 1H), 8.22 (d, *J* = 5.0 Hz, 1H), 8.16 (dd, *J* = 4.8, 1.4 Hz, 1H), 8.00 - 7.93 (m, 1H), 7.61 - 7.55 (m, 2H), 7.44 - 7.39 (m, 2H), 7.36 (dd, *J* = 8.3, 4.8 Hz, 1H), 7.00 (*d, J* = 5.0 Hz, 1H), 3.87 - 3.75 (m, 3H), 3.14 - 3.03 (m, 2H), 2.17 - 2.03 (m, 2H), 1.79 - 1.64 (m, 2H) | 492 |
| **28** | 1-(1-(3-Chloro-4-((4-(trifluoromethoxy)b enzyl)oxy)pyridin-2-yl)piperidin-4-yl)-3-(pyridin-3-yl)urea | 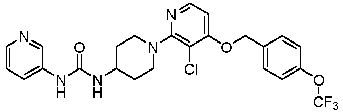 | (DMSO-*d₆*) δ 8.54 (d, *J* = 2.4 Hz, 2H), 8.28 - 8.05 (m, 2H), 7.97 - 7.82 (m, 1H), 7.70 - 7.55 (m, 2H), 7.43 (*d, J* = 8.2 Hz, 2H), 7.25 (dd, *J* = 8.4, 4.7 Hz, 1H), 6.94 (d, *J* = 5.7 Hz, 1H), 6.41 (d, *J* = 7.7 Hz, 1H), 5.32 (s, 2H), 3.84 - 3.66 (m, 1H), 3.60 (dd, *J* = 13.1, 4.0 Hz, 2H), 3.04 - 2.81 (m, 2H), 2.08 - 1.81 (m, 2H), 1.68 - 1.48 (m, 2H) | 522 |
| **29** | 1-(1-(3-Chloro-4-((3-(trifluoromethoxy)b enzyl)oxy)pyridin-2-yl)piperidin-4-yl)-3-(pyridin-3-yl)urea | 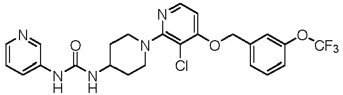 | (DMSO-*d₆*) δ 8.54 (d, *J* = 2.7 Hz, 2H), 8.19 - 8.03 (m, 2H), 7.94 - 7.84 (m, 1H), 7.57 (t, *J* = 7.9 Hz, 1H), 7.53 - 7.43 (m, 2H), 7.36 (d, *J* = 8.0 Hz, 1H), 7.25 (dd, *J* = 8.3, 4.7 Hz, 1H), 6.93 (d, *J=* 5.7 Hz, 1H), 6.41 (d, *J=* 7.7 Hz, 1H), 5.35 (s, 2H), 3.79 - 3.65 (m, 1H), 3.65 - 3.52 (m, 2H), 3.05 - 2.83 (m, 2H), 2.01 - 1.84 (m, 2H), 1.72 - 1.45 (m, 2H) | 522 |

### II. Biological Evaluation

Compounds were tested to assess their ABHD12 activity using the following assay. ***In vitro* competitive activity-based protein profiling**
Mouse brain membrane proteomes (50 µL, 1.0 mg/mL total protein concentration) were preincubated with varying concentrations of inhibitors at 37 °C. After 30 min, FP-Rhodamine probe (1.0 µL, 50 µM in DMSO) was added and the mixture was incubated for 30 min at room temperature. Reactions were quenched with SDS loading buffer (15 µL - 4X) and proteins were resolved by SDS-PAGE gel (10% acrylamide). Following in-gel fluorescence imaging, enzyme activity was determined by measuring intensity of gel bands corresponding to ABHD12 using ImageJ software. Percent enzyme inhibition at 1 µM and IC₅₀ data from this assay is shown in Table 1.

**TABLE 1**

| **Ex** | **ABHD12 % Inhibition 1 µM (mouse)** | **ABHD12 IC₅₀ (mouse)** |
|---|---|---|
| **1** | B (at 10 µM) | |
| **2** | A | ** |
| **3** | D | |
| **4** | C | |
| **5** | C | |
| **6** | A | ** |
| **7** | C | |
| **8** | B (at 10 µM) | |
| **9** | A | ** |
| **10** | A | *** |
| **11** | A (at 50 µM) | |
| **12** | C (at 50 µM) | |
| **13** | C | |
| **14** | A | ** |
| **15** | A | ** |
| **16** | A | ** |
| **17** | B | |
| **18** | D | |
| **19** | B (at 50 µM) | |
| **20** | B | |
| **21** | D | |
| **22** | C (at 50 µM) | |
| **23** | A | ** |
| **24** | C | |
| **25** | D | |
| **26** | A | ** |
| **27** | A | ** |
| **28** | C | |
| **29** | C | |

| | | |
|---|---|---|
| *** IC₅₀ is less than or equal to 100 nM; ** IC₅₀ is greater than 100 nM and less than 1 µM; * IC₅₀ is greater than or equal to 1 µM and less or equal to 25 µM. A = % inhibition is greater than or equal to 75%; B = % inhibition is greater than or equal to 50% and less than 75%; C = % inhibition is greater than or equal to 25% and less than 50%; D = % inhibition is greater than or equal to 0% and less than 25%. | | |

## Claims

1. A compound selected from: 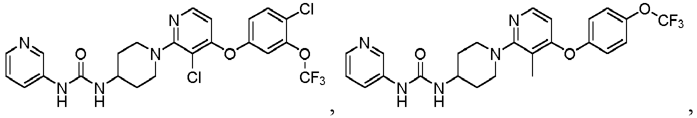 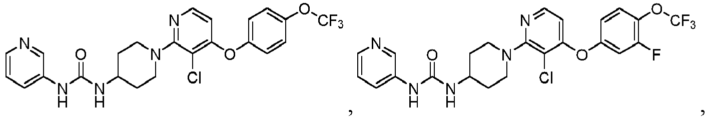 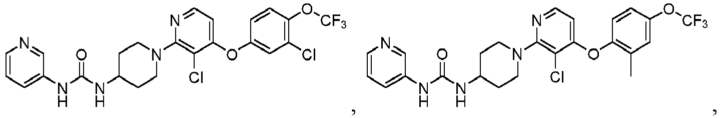 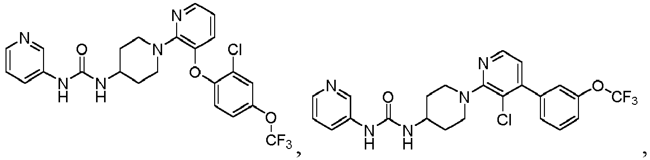 and 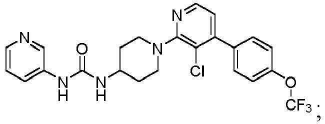 or a pharmaceutically acceptable salt thereof.

2. A pharmaceutical composition comprising a compound of claim 1, or a pharmaceutically acceptable salt thereof, and at least one pharmaceutically acceptable excipient.

## Patentansprüche

1. Verbindung, ausgewählt aus: 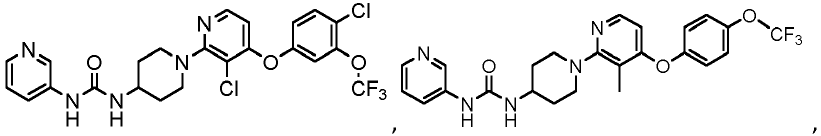 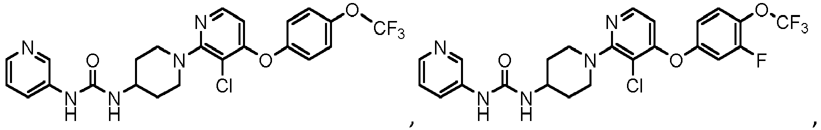 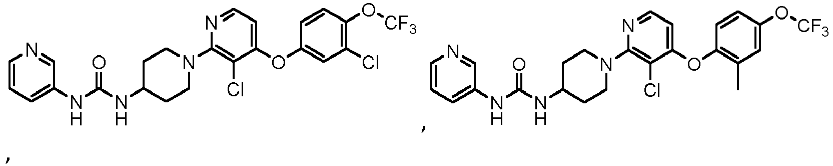 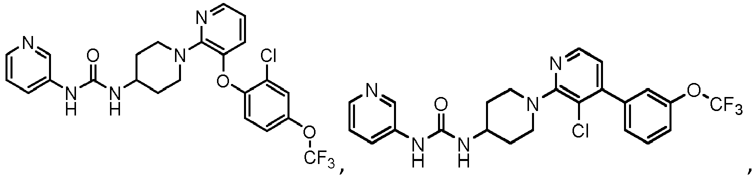 und 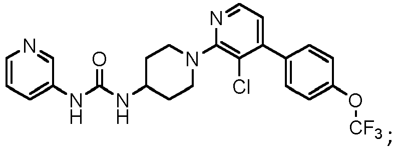 oder ein pharmazeutisch akzeptables Salz davon.

2. Pharmazeutische Zusammensetzung, die eine Verbindung nach Anspruch 1 oder ein pharmazeutisch akzeptables Salz davon und mindestens einen pharmazeutisch akzeptablen Hilfsstoff umfasst.

## Revendications

1. Composé sélectionné parmi : 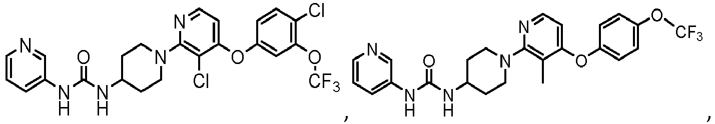 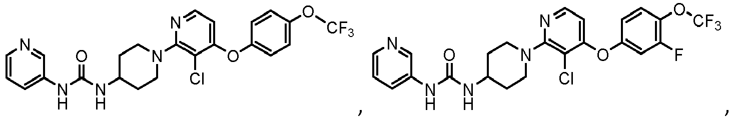 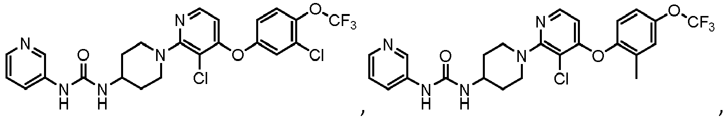 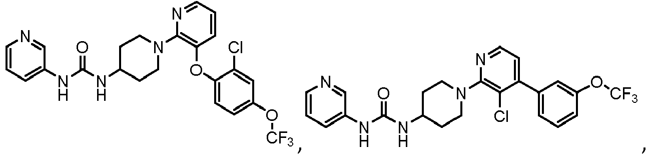 et 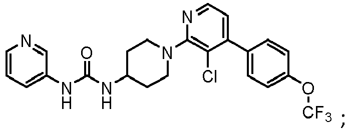 ou un sel pharmaceutiquement acceptable de celui-ci.

2. Composition pharmaceutique comprenant un composé selon la revendication 1, ou un sel pharmaceutiquement acceptable de celui-ci, et au moins un excipient pharmaceutiquement acceptable.
